# FASCICULE DE BREVET EUROPEEN

(11) **EP 4 423 754 B1**
(45) Date de publication et mention de la délivrance du brevet: **23.07.2025**
(21) Numéro de dépôt: 22813436.7
(22) Date de dépôt: 28.10.2022
(51) Int. Cl.: G16H 10/40, G16H 30/20, G16H 30/40, G01B 9/02, G01N 21/64, G06T 7/00

(54) **SYSTÈMES DE CARACTÉRISATION D'UNE RÉGION D'INTÉRÊT D'UN TISSU BIOLOGIQUE**
SYSTEME ZUR CHARAKTERISIERUNG EINES INTERESSENBEREICHS EINES BIOLOGISCHEN GEWEBES
SYSTEMS FOR CHARACTERIZING A REGION OF INTEREST OF A BIOLOGICAL TISSUE

(30) Priorité: 29.10.2021 FR 2111545
(43) Date de publication de la demande: 04.09.2024
(73) Titulaire: Damae Medical, 75013 Paris (FR)
(72) Inventeur: OGIEN, Jonas, 18000 Bourges (FR); DAURES, Anthony, 92150 Suresnes (FR)
(74) Mandataire: Osha BWB
(86) Numéro de dépôt international: PCT/EP2022/080301
(87) Numéro de publication internationale: WO 2023/073220

(56) Documents cités:
- FR-A1- 3 107 604
- US-B2- 9 185 357
- DAVID ARTHUR ET AL: "Line-field confocal optical coherence tomography operating simultaneously at 800 nm and 1300 nm center wavelengths", PROGRESS IN BIOMEDICAL OPTICS AND IMAGING, SPIE - INTERNATIONAL SOCIETY FOR OPTICAL ENGINEERING, BELLINGHAM, WA, US, vol. 10890, 4 March 2019 (2019-03-04), pages 108902I - 108902I, XP060117259, ISSN: 1605-7422, ISBN: 978-1-5106-0027-0, DOI: 10.1117/12.2508465
- OGIEN JONAS ET AL: "Video-mosaicking of human skin in vivo using handheld line-field confocal optical coherence tomography", PROGRESS IN BIOMEDICAL OPTICS AND IMAGING, SPIE - INTERNATIONAL SOCIETY FOR OPTICAL ENGINEERING, BELLINGHAM, WA, US, vol. 11211, 19 February 2020 (2020-02-19), pages 1121114 - 1121114, XP060128367, ISSN: 1605-7422, ISBN: 978-1-5106-0027-0, DOI: 10.1117/12.2544897

## Description

### Domaine technique de l'invention

La présente description concerne des systèmes de caractérisation d'une région d'intérêt d'un tissu biologique, et s'applique notamment à la caractérisation d'une région d'intérêt de la peau dans le cadre d'une exérèse.

### Etat de la technique

Dans le cadre par exemple de l'exérèse d'une tumeur de la peau, il est important pour le chirurgien de caractériser au mieux l'étendue de la tumeur afin d'extraire l'intégralité du tissu tumoral tout en limitant l'extraction du tissu sain autour de la tumeur. On appelle généralement « marges chirurgicales » ou « marges d'exérèse » les contours d'une étendue spatiale qui englobe la région du tissu à extraire. Les marges d'exérèse doivent également avantageusement prendre en compte la présence de lésion en profondeur dans la peau.

De façon générale, on connaît des méthodes optiques pour déterminer les marges d'exérèse en chirurgie.

Ainsi par exemple, l'article de A. Alfonso-Garcia *et al.* [Réf. 1] décrit, en neurochirurgie, une technique d'imagerie basée sur une fluorescence multi-photonique *FLIm* (pour « *Fluorescence Liftetime Imaging* ») qui permet d'identifier, en temps réel pendant une intervention chirurgicale, des régions du cerveau atteintes d'une tumeur et les régions saines, afin de délimiter les marges chirurgicales. Plus précisément, la région d'intérêt est filmée au moyen d'une caméra. Une analyse de fluorescence *FLIm* est effectuée en parallèle au moyen d'une fibre optique déplacée par un opérateur au niveau de la région d'intérêt, la fibre optique permettant le transport du faisceau lumineux d'excitation ainsi que la collection du signal de fluorescence. Un paramètre caractéristique de l'analyse par fluorescence *FLIm,* à savoir la durée de vie de fluorescence codée en couleur, est superposé aux images vidéo afin de présenter à l'opérateur une vue en réalité augmentée de la région d'intérêt.

Une telle technique est cependant difficilement adaptable à l'exérèse de tumeurs de la peau du fait de la précision requise pour la délimitation des marges d'exérèse, d'une part car il n'y a pas de résolution en profondeur du signal de fluorescence dans la technique décrite ci-dessus et d'autre part car il peut y avoir des erreurs de positionnement entre les images de surface acquises par la caméra et la représentation du paramètre caractéristique de la fluorescence, du fait de bougés inévitables du tissu biologique à analyser.

Dans la demande de brevet publiée WO2020087164 [Réf. 2], des images de tomographie par cohérence optique ou images OCT pour « *optical Coherence Tomography* » sont utilisées pour délimiter les marges d'exérèse de tissus ablatés, par exemple pour déterminer si une tumeur a été intégralement extraite, notamment dans les interventions de chirurgie mammaire conservatrice. Un réseau de neurones convolutif ou CNN pour « *Convolutional neural network* » est formé pour reconnaitre dans les images OCT une probabilité de présence d'anomalies dans la région explorée. Les images OCT peuvent être tridimensionnelles, ce qui permet de prendre aussi en compte les régions tumorales en profondeur pour la détermination des marges d'exérèse. Une image annotée de l'image tridimensionnelle ainsi acquise peut être générée, avec une indication sur la probabilité d'anomalies dans la région d'intérêt.

Cependant, la méthode décrite dans la [Réf.2] est adaptée à des applications ex vivo et ne serait pas adaptée à des applications *in vivo.* D'une part parce que l'acquisition de l'ensemble des images à partir desquelles l'analyse est faite est longue (typiquement plusieurs minutes). D'autre part parce que même si des outils d'intelligence artificielle sont utilisés pour la reconnaissance d'anomalies dans les images OCT, ces images resteraient difficiles à interpréter pour un praticien dans le cadre d'une caractérisation *in vivo,* lors de laquelle les praticiens sont habitués à observer des images de surface.

La demande de brevet publiée FR 3107604 [Réf.8] décrit un procédé de gestion de blocs de commandes destinés à un système d'imagerie en microscopie configuré pour acquérir des images d'un échantillon.

La demande de brevet publiée US9185357 [Réf. 9] décrit un microscope à sectionnement optique multimodal pour l'imagerie plein champ d'un échantillon volumique et diffusant. L'article de A. Davis *et al.* [Réf. 10] décrit un système d'imagerie basé sur la tomographique par cohérence optique confocale à balayage de ligne.

La présente description propose des systèmes de caractérisation d'une région d'intérêt d'un tissu biologique, permettant en particulier une caractérisation *in vivo* pour guider une exérèse facilement, notamment une caractérisation de la peau, qui permette tout à la fois d'avoir une excellente précision et d'être interprétable facilement par un praticien.

### Résumé de l'invention

Dans la présente description, le terme « comprendre » signifie la même chose que « inclure », « contenir », et est inclusif ou ouvert et n'exclut pas d'autres éléments non décrits ou représentés. En outre, dans la présente description, le terme « environ » ou « sensiblement » est synonyme de (signifie la même chose que) présentant une marge inférieure et/ou supérieure de 10%, par exemple 5%, de la valeur respective.

Selon un premier aspect, la présente description concerne un système de caractérisation d'une région d'intérêt d'un tissu biologique, comprenant :
- un dispositif de visée comprenant :
   - un dispositif d'éclairage plein champ configuré pour éclairer le tissu biologique dans une première bande spectrale ;
   - un détecteur bidimensionnel comprenant une aire de détection;
   - un dispositif d'imagerie plein champ comprenant un premier axe optique et configuré pour conjuguer optiquement une surface élémentaire du tissu biologique avec ladite aire de détection du détecteur bidimensionnel, le détecteur bidimensionnel produisant, en fonctionnement, une image en réflexion de ladite surface élémentaire;
- un dispositif d'analyse microscopique comprenant :
   - un objectif de microscope comprenant un deuxième axe optique, solidaire mécaniquement du premier axe optique;
   - une voie d'éclairage configurée pour éclairer le tissu biologique selon un premier motif d'éclairage inclus dans ladite surface élémentaire, et dans une deuxième bande spectrale;
   - une voie de détection comprenant ledit objectif de microscope, ladite voie de détection étant configurée pour détecter selon un motif de détection inclus dans ladite surface élémentaire, un faisceau lumineux émis par le tissu biologique en réponse audit éclairage du tissu biologique et générer une information d'analyse microscopique;
- une unité de traitement de données comprenant
   - un premier module de traitement configuré pour déterminer à partir de ladite information d'analyse microscopique au moins un premier paramètre de caractérisation du tissu biologique en un nombre de points donné dudit motif de détection ;
   - un deuxième module de traitement configuré pour
      - repérer, par rapport à une image de surface de la région d'intérêt, chaque image de surface élémentaire d'une pluralité d'images de surface élémentaires acquises successivement par déplacement du dispositif d'imagerie plein champ du dispositif de visée et
      - produire au moins un premier élément de cartographie dudit au moins un premier paramètre de caractérisation, à partir dudit au moins un premier paramètre de caractérisation déterminé pour au moins une partie des images de surface élémentaires de la dite pluralité d'images de surface élémentaires;
- un module d'affichage configuré pour afficher une cartographie du tissu comprenant au moins ledit premier élément de cartographie, ladite cartographie étant repérée par rapport à ladite image de surface de la région d'intérêt.

Dans la présente description, le motif d'éclairage dépend de la voie d'éclairage de la voie d'analyse microscopique et peut comprendre un point d'éclairage, une ligne d'éclairage ou une surface d'éclairage, par exemple une surface rectangulaire résultant du balayage d'un point d'éclairage ou d'une ligne d'éclairage. Un point d'éclairage est défini plus précisément comme le motif de diffraction résultant de la focalisation, par l'objectif de microscope de la voie d'analyse microscopique, d'un faisceau de lumière collimaté incident sur ledit objectif. Le motif d'éclairage peut également comprendre une surface d'éclairage qui ne résulte pas d'un balayage, par exemple une surface à géométrie circulaire, dans le cas d'une voie d'analyse microscopique plein champ.

Le faisceau lumineux émis par le tissu biologique en réponse à l'éclairage de l'échantillon peut être un faisceau réfléchi, rétrodiffusé, ou résultant d'un processus d'émission à une autre longueur d'onde (par exemple fluorescence, diffusion Raman, etc.)

Par ailleurs, une surface élémentaire est définie par le champ dans l'espace objet du dispositif d'imagerie plein champ du dispositif de visée, appelé « champ effectif » dans la présente description. Une surface élémentaire est par exemple définie par un cercle lorsque par exemple le champ effectif est limité par les optiques du dispositif de visée, ou par un rectangle lorsque le champ effectif est limité par l'aire de détection du détecteur bidimensionnel du dispositif de visée. Une image d'une surface élémentaire produite par le dispositif de visée est également appelée « image de visée » dans la présente description. Le motif de détection est inclus dans la surface élémentaire produite par le dispositif de visée et est inclus dans le motif d'éclairage ou est du même ordre de grandeur, et dépend de la voie de détection du dispositif d'analyse microscopique. Le motif de détection peut comprendre un point de détection, une ligne de détection ou une surface de détection, par exemple une surface rectangulaire résultant du balayage d'une ligne, ou, dans le cas d'une voie d'analyse microscopique plein champ, une surface conjuguée optiquement avec une aire de détection d'un détecteur. Un point de détection est ici défini dans l'espace objet par une zone élémentaire conjuguée optiquement avec un détecteur élémentaire d'un détecteur de la voie de détection du dispositif d'analyse microscopique.

Le système de caractérisation selon le premier aspect, du fait de la combinaison originale des caractéristiques, permet un repérage d'au moins un premier paramètre de caractérisation du tissu, en un nombre de points donné dudit motif de détection, par rapport à une image de surface de la région d'intérêt, avec une excellente précision, c'est-à-dire à mieux que quelques microns.

Un premier élément de cartographie dudit au moins un premier paramètre de caractérisation peut alors être produit à partir du premier paramètre de caractérisation du tissu repéré en un nombre de points donné de l'image de surface de la région d'intérêt. Cet élément de cartographie peut directement correspondre à une association de caractéristiques visuelles données (couleur, opacité, épaisseur ...) pour ces points ou une partie de ces points. Cet élément de cartographie peut également résulter d'une application d'opérations linéaires (interpolation, moyennage ...), en particulier de manière à former une surface continue, associée à des caractéristiques visuelles données (couleur, opacité, contour, netteté du contour ...). Cet élément de cartographie peut également résulter d'opérations morpho-mathématiques (dilatation, érosion ...) appliqué à la surface continue, et associé à des caractéristiques visuelles données (couleur, opacité, contour, netteté du contour ...).

Selon un ou plusieurs exemples de réalisation, lesdites images de surface élémentaires présentent un recouvrement partiel et ladite image de surface de la région d'intérêt est produite par mosaïquage de ladite pluralité d'images de surface élémentaires. Le repérage d'une image d'une surface élémentaire dans l'image de surface de la région d'intérêt est alors automatiquement réalisé.

Par exemple, ladite combinaison par mosaïquage est réalisée au fur et à mesure de l'acquisition des images desdites surfaces élémentaires, ce qui permet d'avoir par exemple un affichage en temps réel de l'image de surface acquise et de la cartographie dudit au moins un premier paramètre d'identification, permettant par exemple à un praticien de prendre des décisions pendant l'acquisition.

Selon un ou plusieurs exemples de réalisation, ledit au moins un premier élément de cartographie est produit au fur et à mesure de l'acquisition.

Dans d'autres exemples de réalisation, ladite combinaison par mosaïquage est établie après acquisition de l'ensemble des images de surfaces élémentaires (images de visée). Il y a alors moins de ressources de traitement utilisées en temps réel.

Selon un ou plusieurs exemples de réalisation, ladite image de surface de la région d'intérêt est produite au moyen d'un système d'imagerie indépendant dudit système de caractérisation. Par exemple, l'image de surface de la région d'intérêt est une image dermoscopique acquise par un dermatoscope.

Dans ces exemples, le repérage d'une image d'une surface élémentaire par rapport à l'image de surface de la région d'intérêt peut être obtenu par des techniques connues, comme par exemple une technique comprenant une identification de points d'intérêts ("*feature-detection*") puis une mise en correspondance de points d'intérêts ("*feature-match*")*.* Une telle technique est décrite par exemple dans l'article de S. Li [Réf. 3]. Un mosaïquage des images de surface élémentaires n'est alors plus nécessaire pour le repérage.

Dans des exemples de réalisation, le repérage de chaque image de surface élémentaire de la pluralité d'images de surface élémentaires par rapport à l'image de surface de la région d'intérêt peut comprendre des étapes de traitement préalable sur lesdites images, comme par exemple : un filtrage de type rehaussement localisé ou débruitage pour masquer ou faire ressortir des structures d'intérêt, l'application à un ensemble desdites images d'un algorithme de normalisation ou de mise en correspondance d'histogramme, l'application à un ensemble desdites images d'un algorithme de mosaïquage afin d'augmenter le taux de recouvrement avec l'image de surface de la région d'intérêt.

Par exemple, dans des exemples de réalisation, le module d'affichage est configuré pour afficher en outre ladite image de surface de la région d'intérêt, l'image de surface de la région d'intérêt étant facilement interprétable par un praticien. Dans d'autres exemples de réalisation, par exemple lorsque l'image de surface est obtenue par mosaïquage de la pluralité des images de surface élémentaires, le module d'affichage peut être configuré pour afficher en outre une autre image de surface de l'ensemble de la région d'intérêt, acquise par un autre système d'imagerie, par exemple une image dermoscopique acquise par un dermatoscope, et repérée par rapport à l'image de surface de la région d'intérêt acquise par le système de caractérisation selon le premier aspect.

Par exemple, dans des exemples de réalisation, le module d'affichage est configuré pour que ladite cartographie du tissu soit affichée de façon superposée à ladite image de surface de la région d'intérêt ou, par exemple lorsque l'image de surface est obtenue par mosaïquage de la pluralité des images de surface élémentaires, de façon superposée à une autre image de surface de l'ensemble de la région d'intérêt, acquise par un autre système d'imagerie et repérée par rapport à l'image de surface de la région d'intérêt acquise par le système de caractérisation selon le premier aspect.

Dans d'autres exemples de réalisation, le module d'affichage peut être configuré pour que ladite cartographie du tissu soit affichée de façon juxtaposée à ladite image de surface de la région d'intérêt ou, par exemple lorsque l'image de surface est obtenue par mosaïquage de la pluralité des images de surface élémentaires, de façon juxtaposée à une autre image de surface de l'ensemble de la région d'intérêt, acquise par un autre système d'imagerie et repérée par rapport à l'image de surface de la région d'intérêt acquise par le système de caractérisation selon le premier aspect. Dans ces exemples de réalisation, le module d'affichage peut être configuré en outre pour représenter à la fois, dans ladite cartographie du tissu et dans l'image de surface de la région d'intérêt, un curseur commun, c'est-à-dire un élément graphique configuré pour repérer la même position spatiale sur la cartographie et ladite image de surface de la région d'intérêt.

Bien entendu, le module d'affichage peut être configuré pour fonctionner dans l'un et/ou l'autre des exemples de réalisation décrits ci-dessus, au choix d'un utilisateur.

Dans tous les cas, une exérèse est facilitée car le praticien peut identifier facilement et précisément, au niveau de la surface du tissu, le contour d'une zone d'intérêt, par exemple le contour d'une tumeur, même non directement visible en surface. Cette précision est rendue possible notamment du fait de la solidarité mécanique entre le premier axe optique du dispositif d'imagerie plein champ du dispositif de visée et le deuxième axe optique de l'objectif de microscope du dispositif d' analyse microscopique.

Selon un ou plusieurs exemples de réalisation, le dispositif d'imagerie plein champ du dispositif de visée comprend ledit objectif de microscope. Dans ces exemples de réalisation, le premier axe optique et le deuxième axe optique sont confondus, ce qui permet d'avoir un système de caractérisation plus compact tout en bénéficiant d'une image de visée de qualité puisqu'elle est obtenue avec l'objectif de microscope. Par ailleurs, le repérage dudit au moins un premier paramètre de caractérisation sur l'image de surface est simplifié.

Dans d'autres exemples de réalisation, le dispositif d'imagerie plein champ du dispositif de visée ne comprend pas l'objectif de microscope du dispositif d'analyse microscopique. Dans ce cas cependant, la solidarité mécanique du premier axe optique et du deuxième axe optique permettra de repérer le motif de détection dans l'image de visée et ainsi de repérer ledit au moins un premier paramètre sur l'image de surface de la région d'intérêt.

Dans des exemples de réalisation, la première bande spectrale du dispositif d'éclairage plein champ du dispositif de visée et la deuxième bande spectrale de la voie d'éclairage du dispositif d'analyse microscopique diffèrent au moins partiellement. Cela permet dans des exemples de réalisation de filtrer au moins partiellement la lumière provenant de la voie d'éclairage du dispositif d'analyse microscopique et incidente dans le dispositif de visée ou inversement, de filtrer au moins partiellement la lumière provenant du dispositif d'éclairage plein champ du dispositif de visée et incidente dans le système d'analyse microscopique.

Selon un ou plusieurs exemples de réalisation, le dispositif d'éclairage plein champ du dispositif de visée comprend une pluralité de sources lumineuses agencées sur une périphérie d'une face distale de l'objectif de microscope, c'est-à-dire la face de l'objectif de microscope dans l'espace du tissu biologique. Cette configuration permet un éclairage direct du tissu biologique. Bien entendu, d'autres configurations sont possibles pour le dispositif d'éclairage plein champ du dispositif de visée.

Selon un ou plusieurs exemples de réalisation, la voie d'éclairage du dispositif d'analyse microscopique est configurée pour éclairer le tissu biologique à travers l'objectif de microscope. Dans d'autres exemples de réalisation, un éclairage direct du tissu biologique est possible, sans passer à travers l'objectif de microscope, par exemple au moyen de fibres optiques.

Selon un ou plusieurs exemples de réalisation, ladite voie d'analyse microscopique est une voie d'imagerie confocale et/ou d'imagerie tomographique par cohérence optique et ladite information d'analyse microscopique du tissu biologique comprend au moins une image du tissu biologique. Par exemple, la voie d'analyse microscopique est une voie d'imagerie tomographique par cohérence optique telle que décrite dans l'état de l'art et est configurée pour former des images *B-Scans, C-Scans* (ou images *en face*) du tissu biologique ou des images 3D du tissu biologique. De façon connue, une image en coupe du tissu biologique *dite* « *B-Scan* », est une image formée dans un plan parallèle à l'axe optique de l'objectif de microscope ; une image en coupe de l'échantillon dite « *C-Scan* », ou image *en face,* est une image formée dans un plan perpendiculaire à l'axe optique de l'objectif de microscope, et une image 3D du tissu biologique résulte de l'acquisition d'une pluralité d'images B-Scans ou d'images C-Scans et permet ainsi une analyse du tissu biologique dans un volume.

Selon un ou plusieurs exemples de réalisation, ladite voie d'analyse microscopique est une voie d'analyse spectroscopique et ladite information d'analyse microscopique du tissu biologique comprend au moins un spectre dudit faisceau lumineux émis par l'échantillon en au moins un point de l'échantillon.

Selon un ou plusieurs exemples de réalisation, ledit au moins un premier paramètre de caractérisation du tissu biologique en fonction duquel le premier module de traitement génère à partir de ladite information d'analyse microscopique une cartographie du tissu est un paramètre choisi parmi : une mesure morphologique, une mesure cytologique, une mesure optique, une mesure caractérisant une composition chimique, une mesure mécanique, une combinaison de ces mesures, un score de caractérisation de l'état du tissu, par exemple basé sur l'une au moins de ces mesures, par exemple un score indiquant une probabilité de présence d'une lésion, par exemple une lésion de nature prédéterminée. Selon un ou plusieurs exemples de réalisation, ledit premier module de traitement de ladite information d'analyse microscopique comprend un module d'intelligence artificielle, par exemple basé sur un modèle d'apprentissage profond (« *deep learning* ») utilisant des réseaux de neurones.

De façon générale, les modules de traitement, unité de traitement ou unité de contrôle auxquels il est fait référence dans la présente description peuvent comprendre une ou plusieurs entités physiques, et se trouver rassembler dans un ou plusieurs ordinateurs. Lorsque dans la présente description, il est fait référence à des étapes de calcul ou traitement pour la mise en œuvre notamment d'étapes de procédés, il est entendu que chaque étape de calcul ou traitement peut être mis en œuvre par logiciel, hardware, firmware, microcode ou toute combinaison appropriée de ces technologies. Lorsqu'un logiciel est utilisé, chaque étape de calcul ou traitement peut être mise en œuvre par des instructions de programme d'ordinateur ou du code logiciel. Ces instructions peuvent être stockées ou transmises vers un support de stockage lisible par l'unité de contrôle et/ou être exécutées par l'unité de contrôle afin de mettre en œuvre ces étapes de calcul ou traitement.

Ainsi, dans un système de caractérisation selon le premier aspect, le premier module de traitement et le deuxième module de traitement de l'unité de traitement peuvent être rassemblés dans un ou plusieurs ordinateurs.

Dans des exemples de réalisation, le tissu biologique est la peau et le système de caractérisation est configuré pour être mis en œuvre in vivo. Le système de caractérisation permet par exemple la caractérisation de lésions cancéreuses.

Selon un ou plusieurs exemples de réalisation, le deuxième module de traitement est configuré, en outre, pour la détermination de marges d'exérèse d'une région d'un tissu à extraire à partir dudit au moins un premier élément de cartographie.

Par exemple, les marges d'exérèses sont déterminées à partir du contour défini par ladite cartographie, par exemple en ajoutant une marge supplémentaire, par exemple une marge supplémentaire comprise entre environ 0,5 mm et 5 environ mm.

Selon un deuxième aspect, la présente description concerne un produit programme d'ordinateur comprenant des instructions de code de programme pour la mise en œuvre d'un procédé de traitement de données en vue de la caractérisation d'une région d'intérêt d'un tissu biologique, lorsque ledit programme est exécuté sur un ordinateur, les données étant obtenues au moyen d'un système d'analyse optique comprenant :
- un dispositif de visée comprenant :
   - un dispositif d'éclairage plein champ configuré pour éclairer le tissu biologique dans une première bande spectrale ;
   - un détecteur bidimensionnel comprenant une aire de détection;
   - un dispositif d'imagerie plein champ comprenant un premier axe optique et configuré pour conjuguer optiquement une surface élémentaire du tissu biologique avec ladite aire de détection du détecteur bidimensionnel, le détecteur bidimensionnel produisant, en fonctionnement, une image en réflexion de ladite surface élémentaire;
- un dispositif d'analyse microscopique comprenant :
   - un objectif de microscope comprenant un deuxième axe optique, solidaire mécaniquement du premier axe optique;
   - une voie d'éclairage configurée pour éclairer le tissu biologique selon un premier motif d'éclairage inclus dans ladite surface élémentaire, et dans une deuxième bande spectrale;
   - une voie de détection comprenant ledit objectif de microscope, ladite voie de détection étant configurée pour détecter selon un motif de détection inclus dans ladite surface élémentaire, un faisceau lumineux émis par le tissu biologique en réponse audit éclairage du tissu biologique et générer une information d'analyse microscopique;
le procédé de traitement de données comprenant les étapes suivantes:
- la détermination à partir de ladite information d'analyse microscopique dudit au moins un premier paramètre de caractérisation du tissu biologique en un nombre de points donné dudit motif de détection ;
- le repérage, par rapport à une image de surface de la région d'intérêt, de chaque image de surface élémentaire d' une pluralité d'images de surface élémentaires acquises successivement par déplacement du dispositif d'imagerie plein champ du dispositif de visée et
- la production d'au moins un premier élément de cartographie dudit au moins un premier paramètre de caractérisation, à partir dudit au moins un premier paramètre de caractérisation déterminé pour au moins une partie des images de surface élémentaires de la dite pluralité d'images de surface élémentaires.

Le système d'analyse optique est par exemple un système tel que décrit dans [Réf. 7].

Le produit programme d'ordinateur peut ainsi être exécuté sur un ordinateur qui ne fait pas partie du système d'analyse optique.

Selon un ou plusieurs exemples de réalisation, lesdites images de surface élémentaires présentent un recouvrement partiel et ladite image de surface de la région d'intérêt est produite par mosaïquage de ladite pluralité d'images de surface élémentaires.

Selon un ou plusieurs exemples de réalisation, la détermination dudit au moins un premier paramètre de caractérisation du tissu biologique est obtenue au moyen d'un module d'intelligence artificielle basé sur un modèle d'apprentissage profond utilisant des réseaux de neurones.

Selon un ou plusieurs exemples de réalisation, le procédé comprend en outre la détermination de marges d'exérèse d'une région d'un tissu à extraire à partir dudit au moins un premier élément de cartographie.

Selon un troisième aspect, la présente description concerne un moyen de stockage lisible par ordinateur et non transitoire, stockant un produit programme d'ordinateur selon le deuxième aspect.

### Brève description des figures

D'autres avantages et caractéristiques de l'invention apparaîtront à la lecture de la description, illustrée par les figures suivantes :
[Fig. 1A], un schéma illustrant un premier exemple d'un système de caractérisation d'une région d'intérêt d'un tissu biologique selon la présente description ;
[Fig. 1B], un schéma illustrant un deuxième exemple d'un système de caractérisation d'une région d'intérêt d'un tissu biologique selon la présente description ;
[Fig. 2], un schéma illustrant un exemple d'un système de caractérisation d'une région d'intérêt d'un tissu biologique mettant en œuvre une analyse microscopique de type imagerie tomographique par cohérence optique ;
[Fig. 3A], un schéma illustrant de façon simplifiée une mise en œuvre d'étapes d'un procédé de caractérisation d'une région d'intérêt d'un tissu biologique, au moyen d'un exemple de système de caractérisation selon la présente description ;
[Fig. 3B], un exemple d'affichage d'une image de surface de la région d'intérêt superposée à une cartographie comprenant une pluralité d'éléments de cartographie respectivement de différents paramètres de caractérisation, obtenues au moyen d'un exemple de procédé selon la présente description ;
[Fig. 3C], un exemple d'affichage d'une image de surface de la région d'intérêt juxtaposée à une cartographie comprenant une pluralité d'éléments de cartographie respectivement de différents paramètres de caractérisation, obtenues au moyen d'un exemple de procédé selon la présente description ;
[Fig. 4A], un schéma représentant un synoptique d'un procédé selon la présente description, selon un premier exemple ;
[Fig. 4B], un schéma représentant un synoptique d'un procédé selon la présente description, selon un deuxième exemple ;
[Fig. 5A], un schéma illustrant une étape d'un procédé de caractérisation tel que décrit sur la Fig. 4A ;
[Fig. 5B], un schéma illustrant une étape d'un procédé de caractérisation tel que décrit sur la Fig. 4A ;
[Fig. 5C], un schéma illustrant une étape d'un procédé de caractérisation tel que décrit sur la Fig. 4A ;
[Fig. 5D], un schéma illustrant une étape d'un procédé de caractérisation tel que décrit sur la Fig. 4A ;
[Fig. 6A], un schéma représentant un synoptique d'un procédé selon la présente description, selon un troisième exemple ;
[Fig. 6B], un schéma représentant un synoptique d'un procédé selon la présente description, selon un quatrième exemple.

### Description détaillée de l'invention

Dans la description détaillée qui suit, de nombreux détails spécifiques sont exposés afin de fournir une compréhension plus approfondie de la présente description. Cependant, il apparaîtra à l'homme du métier que la présente description peut être mise en œuvre sans ces détails spécifiques. Dans d'autres cas, des caractéristiques bien connues n'ont pas été décrites en détail pour éviter de compliquer inutilement la description.

Par ailleurs, sur les figures, les éléments ne sont pas représentés à l'échelle pour une meilleure visibilité.

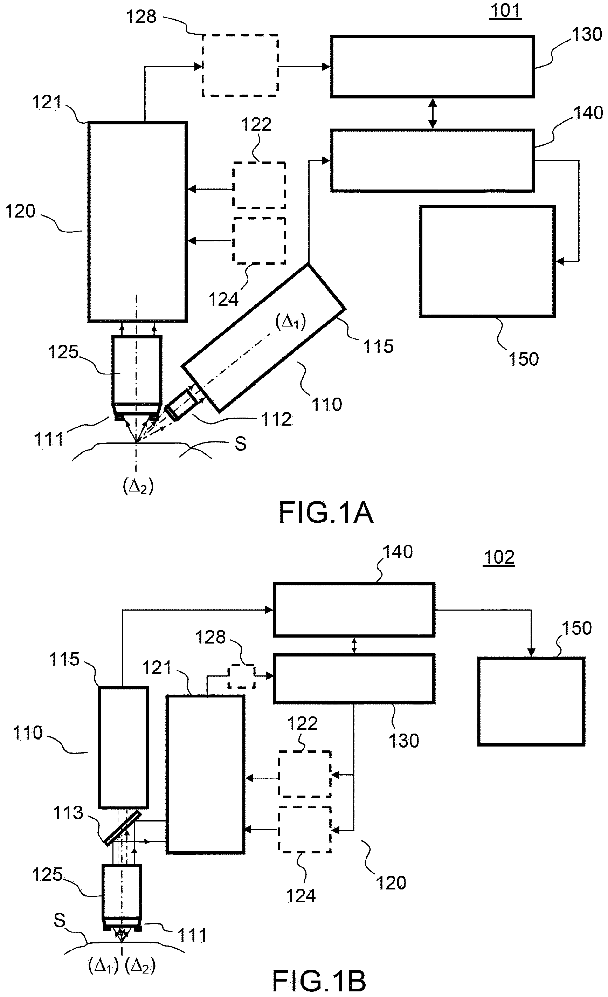

La Fig. 1A représente un schéma illustrant un premier exemple 101 d'un système de caractérisation d'une région d'intérêt d'un tissu biologique S selon la présente description et la Fig. 1B représente un schéma illustrant un deuxième exemple 102 d'un système de caractérisation d'une région d'intérêt d'un tissu biologique selon la présente description. Les systèmes de caractérisation 101, 102 comprennent chacun un dispositif de visée 110 comprenant un dispositif d'éclairage plein champ 111, par exemple un ensemble de diodes électroluminescentes ou LED, configuré pour éclairer le tissu biologique dans une première bande spectrale, un détecteur bidimensionnel (non représenté sur les figures) et un dispositif d'imagerie plein champ (non représenté sur les figures), seul l'axe optique Δ₁ du dispositif d'imagerie plein champ étant schématisé. Le dispositif d'imagerie plein champ du dispositif de visée est configuré pour conjuguer optiquement une surface élémentaire du tissu biologique avec une aire de détection du détecteur bidimensionnel, le détecteur bidimensionnel produisant, en fonctionnement, une image en réflexion de ladite surface élémentaire, appelée « image de visée » dans la présente description. Par exemple, le dispositif de visée 110 comprend une monture 115 configurée pour recevoir tout ou partie des éléments opto-électroniques formant le dispositif d'imagerie plein champ et le détecteur bidimensionnel.

Les systèmes de caractérisation 101, 102 comprennent en outre chacun un dispositif d'analyse microscopique 120 comprenant un objectif de microscope 125 comprenant un axe optique Δ₂, solidaire mécaniquement de l'axe optique Δ₁ du dispositif d'imagerie plein champ.

Dans l'exemple de système de caractérisation 101 illustré sur la Fig. 1A, les axes optiques Δ₁ du dispositif d'imagerie plein champ du dispositif de visée 110 et Δ₂ de l'objectif de microscope 125 sont distincts. La solidarité mécanique des axes Δ₁ et Δ₂ est par exemple assurée par une monture commune (non représentée sur la Fig. 1A).

Dans l'exemple de système de caractérisation 102 illustré sur la Fig. 1B, l'objectif de microscope 125 est compris dans le dispositif d'imagerie plein champ du dispositif de visée 110 et les axes optiques Δ₁ et Δ₂ sont confondus. Dans cet exemple, un élément séparateur de faisceaux 113 permet de séparer les voies de détection du dispositif de visée et du dispositif d'analyse microscopique.

Dans les deux exemples, on peut définir une extrémité distale du système de caractérisation configurée pour se trouver en contact ou à proximité du tissu biologique S. dans le cas par exemple d'une caractérisation de la peau, l'extrémité distale du système de caractérisation peut comprendre une lame de verre (non représentée sur les figures) ou une pièce mécanique (non représenté sur les figures) qui permet de fixer une distance prédéterminée entre la peau et une pupille d'entrée de l'objectif de microscope.

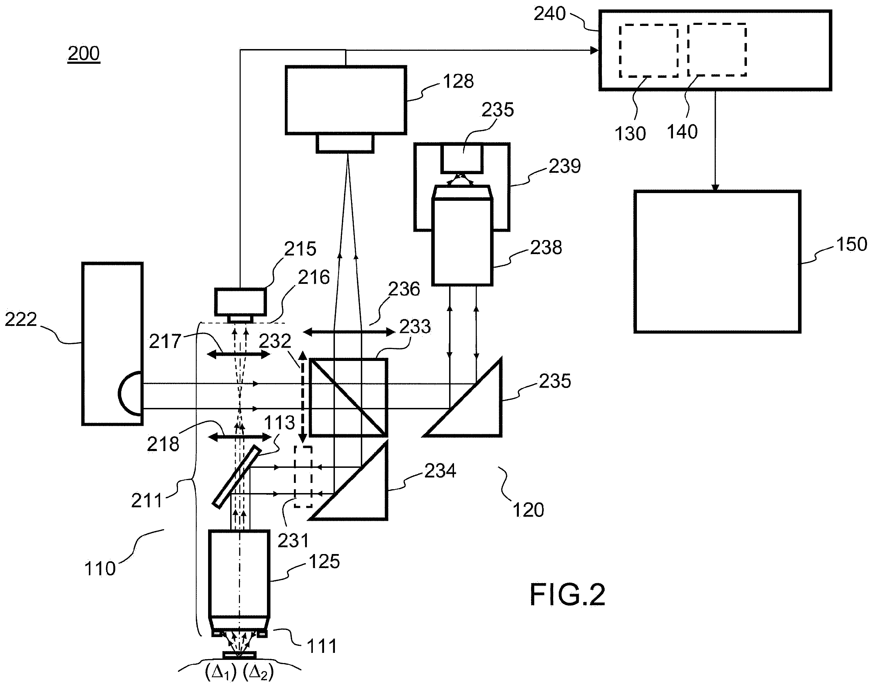

Dans ces exemples, le dispositif d'éclairage plein champ 111 du dispositif de visée comprend un ensemble de LED agencées sur une extrémité distale du dispositif de visée. Le dispositif d'analyse microscopique 120 dans chacun de ces exemples comprend également une voie d'éclairage configurée pour éclairer le tissu biologique, par exemple à travers l'objectif de microscope, selon un premier motif d'éclairage inclus dans ladite surface élémentaire, et une voie de détection comprenant ledit objectif de microscope 125, ladite voie de détection étant configurée pour détecter selon un motif de détection inclus dans ladite surface élémentaire, un faisceau lumineux émis par le tissu biologique en réponse audit éclairage du tissu biologique et générer une information d'analyse microscopique. Une voie d'éclairage et une voie de détection du dispositif d'analyse microscopique 120 sont décrites en relation avec la Fig. 2 dans le cas d'un exemple particulier.

Dans les exemples représentés sur les Fig. 1A et Fig. 1B, le dispositif d'analyse microscopique 120 comprend une monture 121 comprenant généralement au moins une partie des éléments opto-électroniques de la voie de détection du dispositif d'analyse microscopique et peut comprendre au moins une partie des éléments opto-électroniques de la voie d'éclairage. Dans certains exemples de réalisation, les éléments opto-électroniques peuvent comprendre de façon non limitative l'un et ou l'autre des éléments suivants : une source d'éclairage, un montage interférométrique, un détecteur et une unité de traitement associée, un ou plusieurs systèmes optiques permettant d'assurer des conjugaisons optiques entre plans images ou plans pupillaires, un ou plusieurs miroirs de renvoi, un ou plusieurs systèmes de balayage, etc. L'agencement de tels éléments optiques est connu par l'homme du métier et dépend de l'analyse microscopique que l'on cherche à réaliser.

Dans certains exemples de réalisation, une partie desdits éléments opto-électroniques peuvent se situer à l'extérieur de la monture 121, comme par exemple une source d'éclairage 124, par exemple une source laser, un détecteur 128, par exemple une caméra, associé à une unité de traitement (non représentée), permettant de générer à partir d'un signal de détection l'information d'analyse microscopique et, optionnellement, un ou plusieurs contrôleurs 122 pour le contrôle des éléments opto-électroniques agencés dans la monture 121. Le ou les contrôleurs sont par exemple des cartes électroniques configurées pour contrôler un déplacement axial de l'objectif de microscope 125 le cas échéant, ou des miroirs d'un système de balayage, une source lumineuse etc. Le ou les contrôleurs, ainsi que la source 124, peuvent être commandés par une unité de contrôle (non représentée). Lorsque le dispositif d'analyse microscopique 120 est configuré pour l'imagerie confocale et/ou l'imagerie tomographique par cohérence optique, ladite information d'analyse microscopique comprend au moins une image du tissu biologique. Lorsque le dispositif d'analyse microscopique 120 est configuré pour l'analyse spectroscopique, ladite information d'analyse microscopique comprend au moins un spectre dudit faisceau lumineux émis par le tissu biologique éclairé.

A noter que dans d'autres exemples de réalisation, la source d'éclairage de la voie d'éclairage et/ou le détecteur de la voie de détection peuvent être intégrés dans la monture 121.

Dans les exemples illustrés sur les Fig. 1A et Fig. 1B, des montures distinctes 115 et 121 sont représentées. Bien entendu, en pratique, dans des exemples de réalisation, une monture unique peut être prévue pour l'agencement des éléments du dispositif de visée et du dispositif d'analyse microscopique.

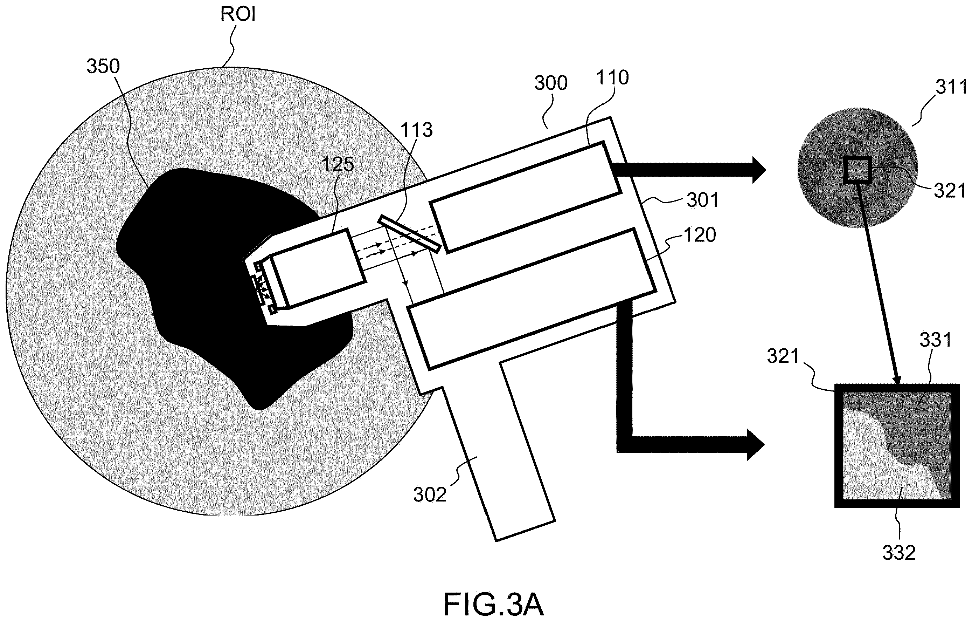

Dans des exemples de réalisation, comme cela est illustré par exemple sur la Fig. 3A, une monture additionnelle (non représentée sur les Fig. 1A et Fig. 1B) peut également être prévue pour assurer la solidarité mécanique entre les axes optiques en solidarisant les montures 121, 115, l'objectif de microscope 125 et le dispositif d'imagerie plein champ du dispositif de visée.

Les systèmes de caractérisation 101, 102 comprennent en outre une unité de traitement avec un premier module de traitement 130 et un deuxième module de traitement 140. Le premier module de traitement 130 est configuré pour déterminer à partir de ladite information d'analyse microscopique au moins un premier paramètre de caractérisation du tissu biologique en un nombre de points donné dudit motif de détection. Le deuxième module de traitement 140 est configuré pour repérer, par rapport à une image de surface de la région d'intérêt, chaque image de surface élémentaire d'une pluralité d'images de surface élémentaires acquises successivement par déplacement du dispositif d'imagerie plein champ du dispositif de visée. Par exemple, l'image de surface de la région d'intérêt est obtenue par mosaïquage de la pluralité d'images de surface élémentaires acquises successivement par déplacement du dispositif d'imagerie plein champ du dispositif de visée et présentant un recouvrement partiel. Le deuxième module de traitement 140 est également configuré pour produire au moins un premier élément de cartographie dudit au moins un premier paramètre de caractérisation, à partir dudit au moins un premier paramètre de caractérisation déterminé pour chaque image de surface élémentaire de ladite pluralité d'images de surface élémentaires.

Les systèmes de caractérisation 101, 102 comprennent par ailleurs un module d'affichage 150 configuré pour afficher une cartographie du tissu comprenant au moins ledit premier élément de cartographie, ladite cartographie étant repérée par rapport à ladite image de surface, comme cela sera expliqué plus en détails par la suite.

Dans l'exemple de la Fig. 1B, le premier axe optique et le deuxième axe optique étant confondus, le repérage de la cartographie dudit au moins un paramètre de caractérisation sur l'image de surface est simplifié. Cependant, dans l'exemple de la Fig. 1A, la solidarité mécanique du premier axe optique et du deuxième axe optique permet de repérer le motif de détection dans l'image de visée et ainsi de repérer la cartographie dudit au moins un paramètre de caractérisation sur l'image de surface de la région d'intérêt.

La Fig. 2 représente un schéma illustrant un exemple d'un système de caractérisation d'une région d'intérêt d'un tissu biologique mettant en œuvre une analyse microscopique de type imagerie tomographique par cohérence optique, par exemple une voie de type LC-OCT telle que décrite par exemple dans la [Réf.4].

Le système de caractérisation 200 comprend dans cet exemple un objectif de microscope 125 commun à un dispositif d'analyse microscopique 120 et à un dispositif d'imagerie grand champ 211 d'un dispositif de visée 110. Dans cet exemple, la ou les montures ne sont pas représentées. Le système de caractérisation 200 comprend comme dans les exemples précédents les modules de traitement 130, 140 par exemple des modules d'un ordinateur 240, ainsi que le module d'affichage 150.

Le dispositif de visée 110 comprend ainsi dans cet exemple l'objectif de microscope 125, l'élément séparateur de faisceaux 113, un dispositif d'éclairage plein champ 111 configuré pour éclairer le tissu biologique dans une première bande spectrale, un détecteur bidimensionnel 215 avec une aire de détection 216, et un ou plusieurs éléments d'imagerie représentés sur la Fig. 2 par les éléments 217, 218 et configurés pour former, avec ledit objectif de microscope 125, un dispositif d'imagerie plein champ 211 qui conjugue optiquement un champ effectif donné de l'échantillon avec l'aire de détection 216 du détecteur bidimensionnel 215. Le dispositif de visée permet ainsi de former une image de visée en réflexion de surface d'une surface élémentaire dont les dimensions sont définies par le champ effectif. Le détecteur bidimensionnel 215 est relié au module de traitement 140 configuré pour combiner par mosaïquage une pluralité d'images de surface élémentaires.

Dans cet exemple, le dispositif d'éclairage plein champ 111 comprend une pluralité de sources lumineuses agencées sur une partie distale du système de caractérisation 200, par exemple sur une périphérie d'une face distale de l'objectif de microscope 125, le dispositif d'éclairage plein champ permettant un éclairage direct du tissu biologique S. Les sources lumineuses sont par exemple des diodes électroluminescentes émettant à des longueurs d'onde comprises entre environ 400 nm et environ 800 nm. Bien entendu, d'autres dispositifs d'éclairage sont possibles, comme par exemple une source agencée en amont de l'objectif de microscope et un élément séparateur de faisceau, par exemple un cube séparateur, configuré pour diriger un faisceau d'éclairage à travers l'objectif de microscope, vers le tissu biologique.

Dans l'exemple de la Fig. 2, le dispositif d'analyse microscopique 120 comprend une voie d'éclairage configurée pour éclairer l'échantillon à travers l'objectif de microscope 125 selon un motif d'éclairage donné dans une deuxième bande spectrale qui peut différer au moins partiellement de la première bande spectrale. La voie d'éclairage comprend par exemple une source d'éclairage 222, configurée par exemple pour l'émission d'un faisceau lumineux collimaté, une lentille ou miroir de renvoi cylindrique 232 (par exemple dans le cas d'une voie de type LC-OCT), un élément séparateur 233 (cube séparateur ou lame séparatrice) et un élément réfléchissant 234 (optionnel) configuré pour envoyer un faisceau d'éclairage émis par la source d'éclairage vers l'objectif de microscope 125. La voie d'éclairage comprend également dans cet exemple un dispositif de balayage 231 du faisceau d'éclairage configuré pour balayer le faisceau d'éclairage selon une ou deux dimensions et l'élément séparateur 113 configuré pour séparer la voie de visée et la voie d'analyse microscopique.

La source d'éclairage 222 peut comprendre par exemple une source d'émission de lumière cohérente (spatialement), polychromatique, collimatée. Des optiques et/ou filtres spatiaux (non représentés) peuvent rendre la source collimatée et/ou cohérente et/ou avec une répartition spectrale spécifique. La longueur d'onde centrale de la source est fonction de l'application, comprise par exemple entre 600 nm et 1500 nm et la largeur spectrale comprise par exemple entre 50 nm et 250 nm environ. Dans le cas d'une application LC-OCT tel que décrite par exemple dans la Réf. 4, la source d'éclairage 222 peut comprendre par exemple, et de façon non limitative, un laser supercontinuum filtré spectralement par une fibre optique pour une émission autour d'environ 800 nm et collimatée par un miroir parabolique hors axe. Dans le cas d'une application à l'imagerie tomographique plein champ ou FF-OCT (selon l'abréviation de l'expression anglo-saxonne « Full Field OCT »), tel que décrit par exemple dans l'article de E. Beaurepaire et al. [Réf. 5]), la source d'éclairage peut être choisie non cohérente spatialement et comprendre des moyens d'éclairage plein champ de l'échantillon, par exemple un système d'éclairage Köhler. L'élément optique cylindrique 232 est optionnel et permet une microscopie avec éclairage selon une ligne (microscopie « *line-field* »)*.*

Le dispositif de balayage 231 du faisceau d'éclairage peut être configuré pour un balayage unidimensionnel ou bidimensionnel d'un point ou d'une ligne pour former de façon connue une image en coupe de l'échantillon dite « *B-Scan* », c'est-à-dire dans un plan parallèle à l'axe optique de l'objectif de microscope, une image en coupe de l'échantillon dite « C-*Scan* », ou image *en face,* c'est-à-dire dans un plan perpendiculaire à l'axe optique de l'objectif de microscope, ou une image 3D de l'échantillon résultant de l'acquisition d'une pluralité d'images B-Scans ou d'images C-Scans. Comme précédemment, le dispositif de balayage peut comprendre un ou plusieurs éléments de balayage choisis parmi les éléments suivants : miroirs galvanométriques, miroirs polygonaux, systèmes de déflexion électro ou acousto-optique, ou une combinaison de ces différents éléments (dans le cas d'un balayage bi-dimensionnel). Le dispositif de balayage peut aussi inclure des optiques pour conjuguer au moins un desdits éléments de balayage à une pupille d'entrée de l'objectif de microscope 125 afin par exemple d'éviter un vignettage.

La voie de détection du dispositif d'analyse microscopique est configurée pour détecter un faisceau lumineux émis par l'échantillon en réponse audit éclairage de l'échantillon, selon un motif de détection donné. Dans cet exemple particulier (non limitatif), la voie de détection comprend un interféromètre pour la mise en œuvre de la microscopie tomographique par cohérence optique. Plus précisément, l'interféromètre comprend un bras objet avec l'objectif de microscope 125, le dispositif de balayage 231 et des éléments réfléchissants ou partiellement réfléchissants 113, 234, 233 configurés pour envoyer un faisceau émis par l'échantillon S en réponse audit éclairage de l'échantillon vers un détecteur 128.

L'interféromètre de la voie de détection comprend en outre un bras de référence, séparé dans cet exemple du bras objet par le cube séparateur 233, et comprenant de façon connue un objectif de microscope 238 (optionnel), par exemple similaire à l'objectif de microscope 125 pour assurer la compensation de la dispersion, un système de compensation de la dispersion (optionnel, non représenté), un miroir de référence 235, une plateforme 239 (optionnelle) configurée par exemple pour faire déplacer le miroir de référence 235 lorsqu'une modulation du chemin optique sur le bras de référence est requise. La voie de détection comprend en outre dans cet exemple un objectif 236 configuré pour conjuguer optiquement, avec l'objectif de microscope, un plan de l'échantillon S avec une aire de détection du détecteur 128.

Le détecteur 128 comprend dans cet exemple un capteur optique avec une aire de détection, et peut aussi inclure des filtres spatiaux pour la détection confocale, si celle-ci n'est pas assurée par les dimensions de l'aire de détection, et/ou des filtres spectraux pour limiter la bande de longueurs d'onde détectée. Le capteur peut comprendre une surface élémentaire de détection (e.g. une photodiode) dans le cas d'un système à balayage ponctuel, un capteur unidimensionnel (e.g. une caméra linéaire) dans le cas d'un système « *line-field* », ou un capteur bidimensionnel dont seule une région d'intérêt est considérée afin de faire office d'aire de détection élémentaire ou de capteur unidimensionnel. Dans le cas d'une application FF-OCT, un capteur bidimensionnel peut être utilisé de façon conventionnelle.

En fonctionnement, des interférences sont créées au niveau de l'aire de détection du détecteur 128 entre la lumière issue du bras de référence et la lumière rétrodiffusée par l'échantillon éclairé selon le motif d'éclairage, éventuellement et de façon connue avec une modulation de la différence de marche entre le bras de référence et le bras objet de l'échantillon, pour la formation d'images tomographiques, notamment d'images *en face.* Une unité de traitement (non représentée) reçoit, de façon connue, des signaux de détection générés par le détecteur 128 et résultant de la détection d'interférences et est configurée pour la reconstitution d'images microscopiques à partir des signaux de détection, par exemple des images en coupe 2D (B-Scan ou C-Scan). L'unité de traitement peut être relié à une unité de stockage (non représenté) des images et/ou vidéos générées. L'unité de traitement est connectée au module de traitement 130 configuré pour générer à partir des images tomographiques une cartographie du tissu en un nombre de points donné dudit motif de détection en fonction d'au moins un paramètre de caractérisation du tissu biologique, comme cela sera expliqué plus en détails par la suite.

Un tel dispositif d'analyse microscopique 120 fonctionne ainsi comme une voie de microscopie tomographique par cohérence optique connue de l'état de l'art.

Bien qu'un exemple particulier soit représenté sur la Fig. 2, l'homme du métier comprendra que le système de caractérisation selon la présente description s'applique à tout montage connu de l'état de l'art pour la microscopie tomographique par cohérence optique, les éléments optomécaniques représentés sur la Fig. 2 pouvant être adaptés en conséquence ou plus généralement à tout montage connu pour l'analyse microscopique d'un tissu biologique.

La Fig. 3A représente un schéma illustrant de façon simplifiée une mise en œuvre d'étapes d'un procédé de caractérisation d'une région d'intérêt ROI d'un tissu biologique S, autour d'ne région suspecte 350, par exemple une région suspecte visible sur la peau, au moyen d'un système de caractérisation 300 selon la présente description.

Comme dans les exemples précédents, le système de caractérisation 300 comprend généralement un dispositif de visée 110 et un dispositif d'analyse microscopique 120, le dispositif d'imagerie grand champ du dispositif de visée 110 comprenant dans cet exemple l'objectif de microscope 125 du dispositif d'analyse microscopique et les voies de visée et d'analyse microscopique étant séparées par l'élément séparateur 113.

Dans cet exemple, au moins une partie des éléments du dispositif de visée et du dispositif d'analyse microscopique, dont l'objectif de microscope 125 sont compris dans une monture commune 301 équipée d'une poignée 302 et appelée « sonde » dans la description. Comme précédemment, on peut définir une extrémité distale d'une telle sonde destinée à venir en contact ou à proximité du tissu que l'on cherche à caractériser, par exemple la peau.

En fonctionnement, le dispositif de visée 110 permet l'acquisition successive d'images en réflexion de surfaces élémentaires 311 ou « images de visée » de ladite région d'intérêt ROI du tissu biologique S, les images de surfaces élémentaires étant acquises successivement et pouvant présenter, dans des exemples de réalisation, un recouvrement partiel.

Pour chaque image de visée d'une pluralité desdites images de visée ainsi acquises, une analyse microscopique du tissu biologique au niveau de l'image de visée est effectuée au moyen du dispositif d'analyse microscopique 120. Comme décrit précédemment, l'analyse microscopique comprend l'éclairage du tissu biologique, par exemple à travers l'objectif de microscope, selon un premier motif d'éclairage donné inclus dans ladite surface élémentaire et la détection, selon un motif de détection 321 inclus dans ladite surface élémentaire, d'un faisceau lumineux émis par le tissu biologique en réponse audit éclairage du tissu biologique pour produire une information d'analyse microscopique, par exemple une image (non représentée sur la Fig. 3A).

Le procédé de caractérisation comprend alors le traitement de ladite information d'analyse microscopique pour déterminer, pour chaque image de visée, au moins un premier paramètre de caractérisation du tissu biologique en un nombre de points donné dudit motif de détection.

Le paramètre de caractérisation est par exemple et de façon non limitative : une mesure morphologique (par exemple présence de structures cellulaires et tissulaires dans différentes couches du tissu, épaisseur d'une ou plusieurs couches du tissu), une mesure cytologique (par exemple une densité de noyaux de cellules, par exemple une densité des noyaux de kératinocyte, ou une anisotropie du collagène), une mesure optique (densité optique, mesure de fluorescence, mesure multiphotonique), une mesure chimique (mesure Raman, mesure spectroscopique), une mesure mécanique (par exemple élastographie ), ou une combinaison de ces mesures.

Le paramètre de caractérisation peut également comprendre un score basé par exemple sur l'une ou plusieurs des mesures précitées, éventuellement avec une pondération. Par exemple un tel score peut exprimer la présence ou l'absence de lésion au sein du tissu, de manière binaire ou non binaire (par exemple via un pourcentage de probabilité, un chiffre sur une échelle donnée ou un résultat binaire).

Ainsi à titre d'exemple, dans l'exemple de la Fig. 3A, le paramètre de caractérisation exprime la présence 332 ou l'absence 331 de lésion au sein du tissu.

La définition du paramètre de caractérisation peut également résulter selon des exemples à la détection d'une propriété identique à plusieurs profondeurs différentes. Par exemple, un paramètre de caractérisation peut être « probabilité de plus de 70 % qu'il s'agisse d'une lésion, mesurée à une profondeur supérieure à 100 µm ».

A noter que la génération de la décision présence/absence de lésion ou du pourcentage de probabilité peut être effectuée par intelligence artificielle, au moyen d'un module d'intelligence artificielle, par exemple basé sur un modèle d'apprentissage profond (« *deep learning* ») utilisant des réseaux de neurones (« *neural networks* »).

De façon générale, un tel module d'intelligence artificielle peut être utilisé pour déterminer un paramètre de caractérisation sur la base de l'une ou d'une combinaison des mesures précitées.

Ainsi, dans des exemples de réalisation, un modèle d'apprentissage profond, basé sur les réseaux de neurones, peut être utilisé sur des images en coupe (*B-Scans* ou *C-Scans*) pour détecter la présence d'une pathologie, par exemple un Carcinome Basocellulaire (BCC) avec un score compris entre 0 et 1. L'ensemble de ces scores visualisés en face, forment une cartographie des zones pathologiques et des zones saines. Une frontière précise peut être calculée grâce à un seuil délimitant les zones à risques.

Dans un exemple de réalisation, un modèle ResNet-18 développé par Microsoft ^{®} avec une sortie binaire est entraîné à prédire la présence ou l'absence de BCC sur des images en coupe verticale (B-Scans). Le modèle est utilisé pour prédire sur chaque image 3D un score de détection en 0 et 1. Afin de limiter les risques de sous-estimer la lésion, un seuil peut être utilisé pour définir les zones pathologiques. L'enveloppe convexe de cette zone peut être considérée comme étant la zone à enlever pour le praticien.

Dans un autre exemple de réalisation, un modèle EfficientNet-B3 développé par Google ^{®} avec une sortie binaire est entraîné à prédire la présence ou l'absence de BCC sur des images en coupe verticale (*B-Scans*). Le modèle est utilisé pour prédire sur chaque image 3D un score de détection en 0 et 1. Afin de limiter au maximum la zone de chirurgie un seuil peut être utilisé pour définir les zones pathologiques L'enveloppe convexe de cette zone peut être considérée comme étant la zone à enlever pour le praticien.

Par ailleurs, il est tout à fait possible d'avoir plusieurs paramètres de caractérisation en un point (si plusieurs paramètres de caractérisation sont vérifiés en ce point), par exemple « probabilité de plus de 70 % qu'il s'agisse d'une lésion » et « épaisseur de l'épiderme inférieure à 100 µm »).

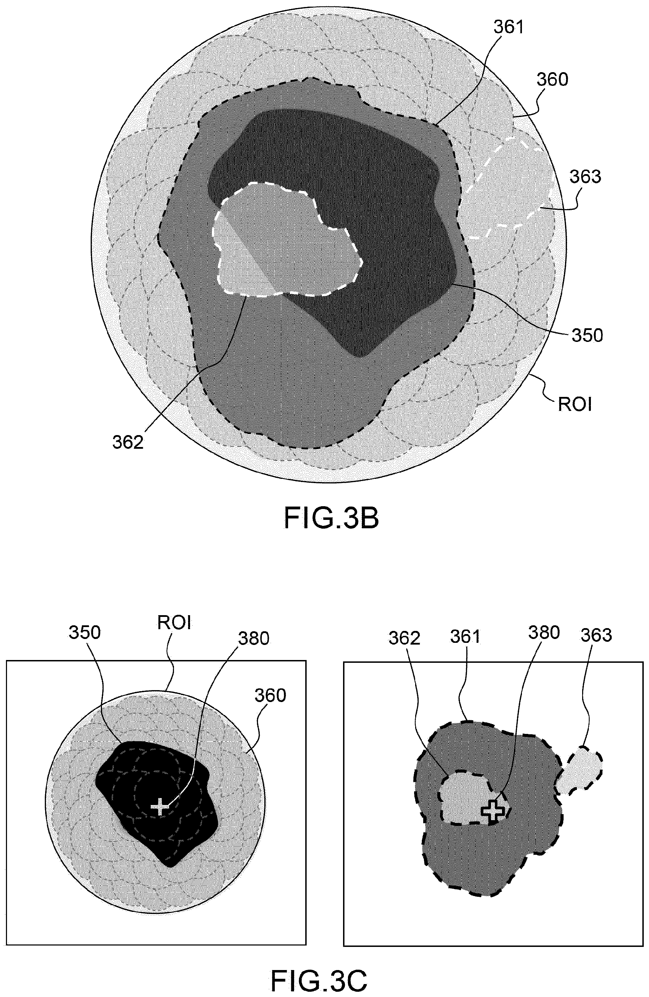

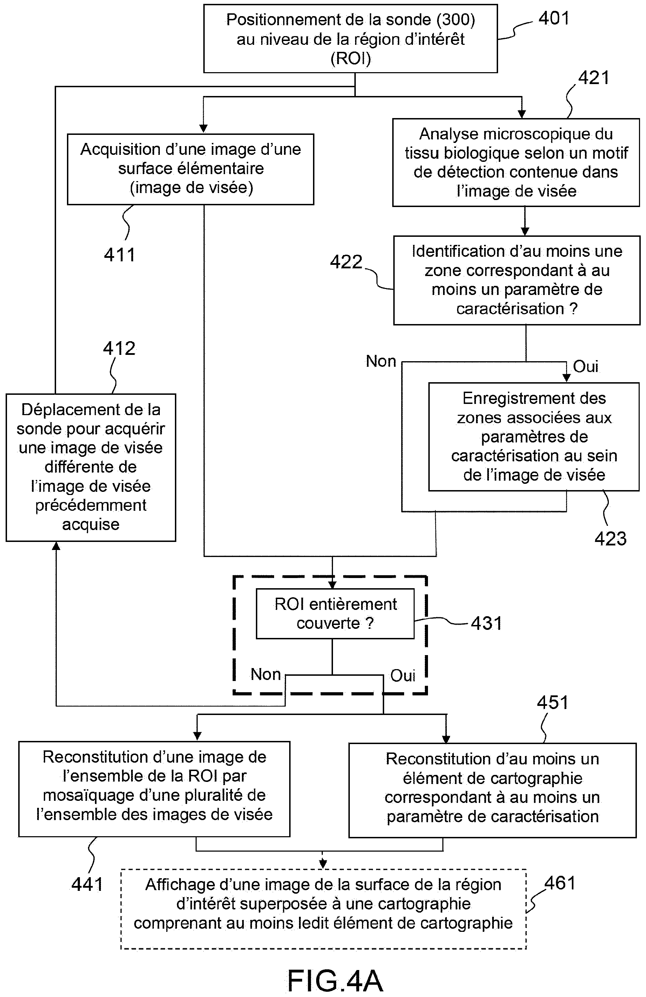

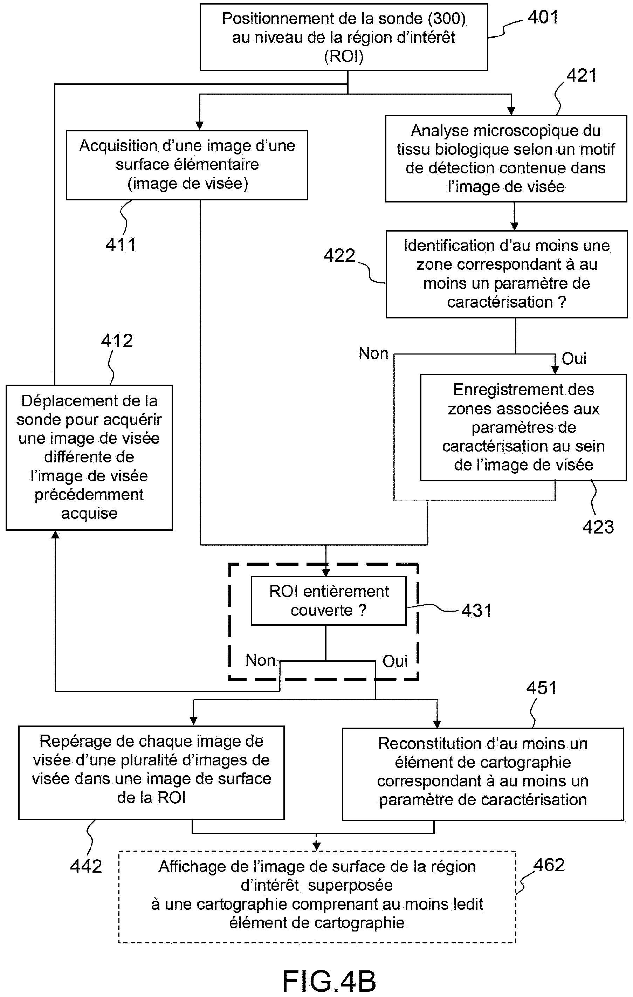

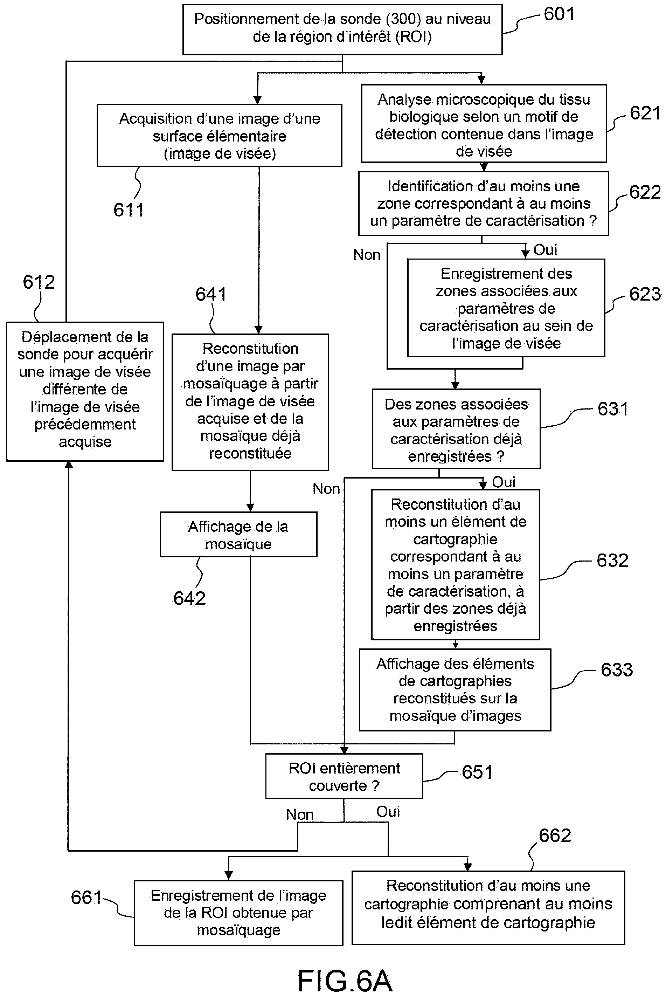

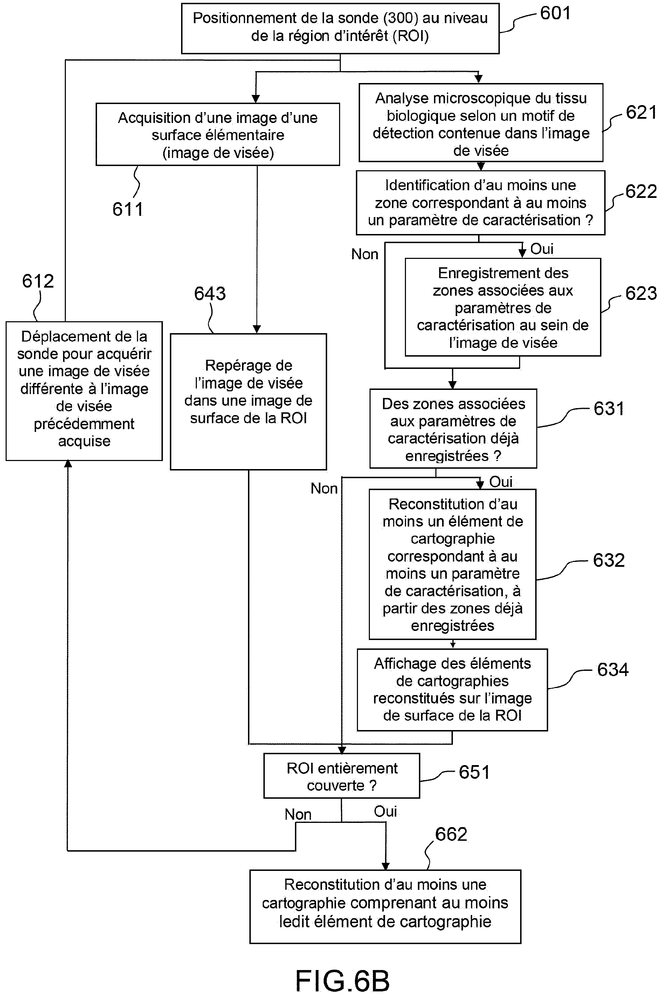

La Fig. 3B illustre ainsi un premier exemple d'affichage d'une image de surface 360 de la région d'intérêt ROI obtenue grâce à un procédé selon la présente description, sur laquelle est visible la région suspecte 350. Des exemples de procédés sont décrits plus en détails au moyen des Fig. 4A, 4B et Fig. 6A, 6B. Superposée à l'image de surface 360, on observe un premier élément de cartographie 361 d'un premier paramètre de caractérisation, un deuxième élément de cartographie 362 d'un deuxième paramètre de caractérisation et un troisième élément de cartographie 363 d'un troisième paramètre de caractérisation.

A noter que dans des exemples de réalisation, l'image de surface de la région d'intérêt (non représentée sur les figures) peut être une image obtenue par mosaïquage d'images de visée ou peut être obtenue au moyen d'un autre système d'imagerie, indépendant. Une telle autre image de surface de la région d'intérêt est par exemple une image dermoscopique obtenue par un dermatoscope.

A noter également que dans le cas notamment où l'image de surface de la région d'intérêt est obtenue par mosaïquage d'images de visée, le ou lesdits éléments de cartographie peuvent être superposées à une autre image de surface, obtenue au moyen d'un autre système d'imagerie, et repérée par rapport à l'image de surface de la ROI obtenue par mosaïquage.

La Fig. 3C illustre un deuxième exemple d'affichage d'une image de surface 360 de la région d'intérêt ROI obtenue grâce à un procédé selon la présente description, sur laquelle est visible la région suspecte 350.

Dans cet exemple, le module d'affichage est configuré pour que la cartographie du tissu selon un ou plusieurs paramètres soit affichée de façon juxtaposée à ladite image de surface de la région d'intérêt. Le module d'affichage est alors configuré pour représenter en outre dans ladite cartographie du tissu et dans ladite image de surface de la région d'intérêt, un curseur commun 380. Le curseur commun peut être un élément graphique configuré pour repérer la même position spatiale sur la cartographie et sur l'image de surface de la région d'intérêt.

Comme précédemment, l'image de surface de la région d'intérêt peut être une image obtenue par mosaïquage d'images de visée ou une image de surface obtenue par un système indépendant, par exemple une image dermoscopique.

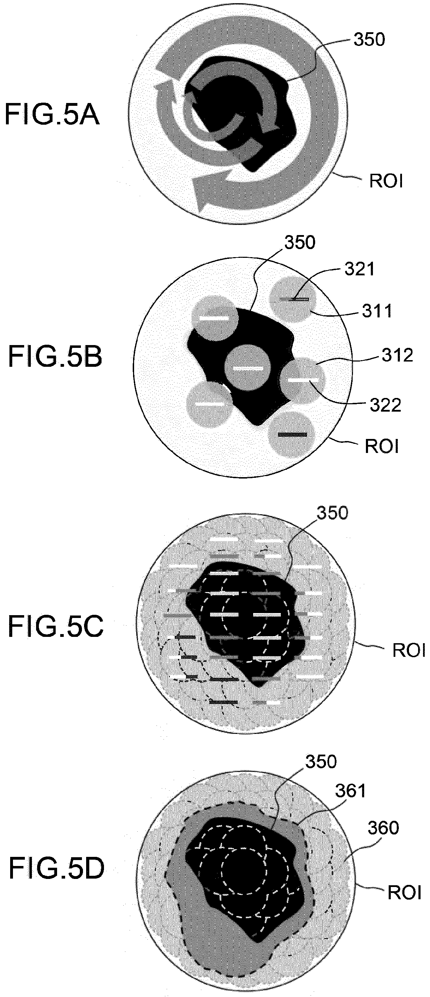

La Fig. 4A illustre de façon détaillée un schéma représentant un synoptique d'un premier exemple de procédé de caractérisation selon la présente description, notamment pour la mise en œuvre du procédé dans une caractérisation *in vivo* d'un tissu biologique. Le procédé est par exemple mis en œuvre avec un système de caractérisation du type de celui décrit en référence aux figures précédentes. Les fig. 5A, Fig. 5B et Fig. 5C illustrent des étapes d'un procédé tel que décrit sur la Fig. 4A.

Comme illustré sur la Fig. 3A, dans le cas où le système de caractérisation forme une sonde 300 pouvant être manipulée par un opérateur, par exemple dans le cadre d'une caractérisation in vivo, le procédé comprend une étape 401 de positionnement de la sonde 300 au niveau de la région d'intérêt ROI du tissu biologique S que l'on cherche à caractériser. Bien entendu, dans une caractérisation ex vivo, un déplacement relatif du système de caractérisation et de l'échantillon peut être fait aussi par un déplacement de l'échantillon.

Le procédé comprend ensuite l'acquisition successive 411, 412 d'images en réflexion de surfaces élémentaires de ladite région d'intérêt du tissu biologique, au moyen d'un dispositif de visée tel que décrit précédemment et comprenant un détecteur bidimensionnel et un dispositif d'imagerie plein champ, les images de surfaces élémentaires acquises successivement présentant un recouvrement partiel.

Par exemple, comme illustré sur la Fig. 4A, le procédé comprend (étape 411) l'acquisition et l'affichage au moyen d'un module d'affichage d'une première image d'une surface élémentaire, appelée « image de visée » dans la présente description. Puis, si la région d'intérêt n'a pas été entièrement couverte (étape 431), l'opérateur peut déplacer la sonde (412) pour image une surface élémentaire différente de la surface élémentaire précédemment imagée par le dispositif de visée.

Comme illustré que la Fig. 5A, le déplacement peut se faire en partant de la région suspecte 350 visible par le praticien puis en s'éloignant de la région 350 pour couvrir toute la région ROI.

Comme illustré sur la Fig. 4A, pour chaque image d'une surface élémentaire d'une pluralité desdites images de surfaces élémentaires ainsi acquises, une analyse microscopique du tissu biologique 421 est réalisée au niveau de ladite surface élémentaire, au moyen d'un dispositif d'analyse microscopique tel que décrit précédemment. L'analyse microscopique comprend notamment l'éclairage du tissu biologique selon un premier motif d'éclairage donné inclus dans ladite surface élémentaire, et la détection, selon un motif de détection inclus dans ladite surface élémentaire, d'un faisceau lumineux émis par le tissu biologique en réponse audit éclairage du tissu biologique pour produire une information d'analyse microscopique.

Le procédé comprend ensuite le traitement de l'information d'analyse microscopique pour déterminer à partir de ladite information d'analyse microscopique au moins un premier paramètre de caractérisation du tissu biologique en un nombre de points donné dudit motif de détection et produire un élément de cartographie du paramètre de caractérisation. Comme illustré sur la Fig. 4A, cette étape peut comprendre plus précisément une étape d'analyse 422 dans laquelle on cherche à identifier au moins une zone de détection du motif de détection pour laquelle l'information d'analyse microscopique correspond à au moins un paramètre de caractérisation. Si c'est le cas, la ou les zones sont enregistrées (étape 423) au sein de l'image de visée correspondante.

La Fig. 5B illustre ainsi par exemple une pluralité d'images de visée (voir par exemple 311, 312) pour lesquelles le motif de détection est une ligne de détection, respectivement indiquée par 321, 322. Les zones blanches et grises représentent respectivement des paramètres de caractérisation différents.

Le procédé comprend ensuite dans cet exemple la combinaison par mosaïquage 441 des images de surface de la pluralité des images de surface pour obtenir une image de surface de la région d'intérêt. La combinaison par mosaïquage peut se faire par une technique connue, comprenant par exemple une étape de recalage (ou « *registration* ») et une étape de combinaison (ou « *stitching* »), de telles techniques étant décrites par exemple dans l'article de J. Ogien et al. [Réf. 6].

La Fig. 5C illustre ainsi une image de surface de la région d'intérêt résultant de la combinaison par mosaïquage des images de visée.

Par ailleurs, au moins un élément de cartographie correspondant à au moins un paramètre est reconstitué (étape 451) à partir des zones enregistrées pour au moins une pluralité des images de visée.

Il est alors possible d'afficher (étape 461) l'image de surface de la région d'intérêt et une cartographie comprenant au moins ledit élément de cartographie, ladite cartographie étant repérée dans ladite image de surface, comme montré sur la Fig. 3B ou sur la Fig. 3C.

La Fig. 5D représente ainsi de façon schématique l'image de surface 360 de la région d'intérêt ROI sur laquelle est superposée un élément de cartographie 361 correspondant par exemple à la région identifiée comme « présentant une lésion », à partir de l'information d'analyse microscopique au niveau de chaque image de visée.

La Fig. 4B illustre un schéma représentant un synoptique d'un deuxième exemple de procédé de caractérisation selon la présente description.

Dans cet exemple, les étapes similaires à celles illustrées au moyen de la Fig. 4A sont repérées par les mêmes références.

Dans cet exemple, le procédé ne comprend plus nécessairement d'étape de mosaïquage d'images de visée. Le procédé comprend ici une étape 442 de repérage de chaque image de visée d'une pluralité d'images de visée dans une image de surface de la région d'intérêt. Cette image de surface de la région d'intérêt peut être une image obtenue par mosaïquage. Il peut s'agir également d'une image obtenue par un système indépendant, par exemple une image dermoscopique obtenue par un dermatoscope. Une étape 462 peut comprendre alors un affichage de l'image de surface de la région d'intérêt superposée à une cartographie comprenant au moins ledit élément de cartographie.

La Fig. 6A illustre de façon détaillée un schéma représentant un synoptique d'un troisième exemple de procédé de caractérisation selon la présente description.

Le procédé comprend comme précédemment une étape 601 de positionnement de la sonde au niveau de la région d'intérêt du tissu biologique que l'on cherche à caractériser.

Le procédé comprend ensuite l'acquisition successive 611, 612 d'images en réflexion de surfaces élémentaires de ladite région d'intérêt du tissu biologique, au moyen d'un dispositif de visée comprenant un détecteur bidimensionnel et un dispositif d'imagerie plein champ, tel que décrit précédemment, les images de surfaces élémentaires acquises successivement présentant un recouvrement partiel.

Dans cet exemple cependant, le procédé comprend après chaque étape 611 d'une image d'une surface élémentaire ou « image de visée », une reconstitution 641 d'une image par mosaïquage à partir de l'image de visée acquise et de la mosaïque reconstituée jusqu'alors au cours du procédé et l'affichage 642 de la mosaïque d'image. Comme précédemment, le mosaïquage est effectué selon des techniques connues, comme celles décrites par exemple dans [Réf. 6].

Puis, si la région d'intérêt n'a pas été entièrement couverte (étape 651), l'opérateur peut déplacer la sonde (612) pour image une surface élémentaire différente de la surface élémentaire précédemment imagée par le dispositif de visée.

Comme illustré sur la FIG. 6A, pour chaque image d'une surface élémentaire d'une pluralité desdites images de surfaces élémentaires acquises, une analyse microscopique 621 du tissu biologique est réalisée au niveau de ladite surface élémentaire, au moyen d'un dispositif d'analyse microscopique tel que décrit précédemment. L'analyse microscopique comprend notamment l'éclairage du tissu biologique selon un premier motif d'éclairage donné inclus dans ladite surface élémentaire, et la détection, selon un motif de détection inclus dans ladite surface élémentaire, d'un faisceau lumineux émis par le tissu biologique en réponse audit éclairage du tissu biologique pour produire une information d'analyse microscopique.

Le procédé comprend ensuite le traitement de l'information d'analyse microscopique pour déterminer à partir de ladite information d'analyse microscopique au moins un premier paramètre de caractérisation du tissu biologique en un nombre de points donné dudit motif de détection et produire, à partir d'une pluralité des images de visée, un élément de cartographie du paramètre de caractérisation. Comme illustré sur la Fig. 6A, cette étape peut comprendre plus précisément une étape d'analyse 622 dans laquelle on cherche à identifier au moins une zone de détection du motif de détection pour laquelle l'information d'analyse microscopique correspond à au moins un paramètre de caractérisation. Si c'est le cas, la ou les zones sont enregistrées (étape 623) au sein de l'image de visée correspondante.

Dans l'exemple de procédé illustré sur la Fig. 6A, au moins un élément de cartographie correspondant à au moins un paramètre est reconstituée à partir des zones enregistrées pour une pluralité des images de visée.

Dans cet exemple cependant, le procédé comprend plus précisément, dans le cas 631 où des zones associées aux paramètres de caractérisation ont déjà été enregistrées, une étape 632 de reconstitution d'au moins un élément de cartographie correspondant à au moins un paramètre de caractérisation, à partir desdites zones enregistrées.

Le procédé comprend en outre (étape 633) l'affichage du ou des élément(s) de cartographie ainsi reconstitués, par exemple de façon superposée à la mosaïque d'images ou alternativement de façon juxtaposée, comme décrit précédemment. Ainsi un utilisateur voit au cours du procédé de caractérisation, la mosaïque d'images se former et croître au fur et à mesure de l'acquisition de nouvelles images de visée, et superposés ou juxtaposés à la mosaïque, le ou les élément(s) de cartographie.

Il est ainsi possible pour un praticien d'avoir une vue d'ensemble de l'acquisition et donc de mieux se repérer par exemple par rapport à une image grand champ précédemment acquise par dermoscopie par exemple. De plus, cette configuration permet potentiellement d'afficher aussi la cartographie en temps réel, et permet donc au praticien de prendre des décisions basées sur la cartographie durant l'acquisition (par exemple de partir dans telle ou telle direction au cours de l'acquisition, ou bien de s'arrêter pour directement extraire une partie du tissu basé sur les informations apportées par la cartographie).

Lorsque l'ensemble de la région a été couverte (étape 651), il est procédé (étape 661) à l'enregistrement de l'image de la région d'intérêt obtenue par mosaïquage au cours du procédé ainsi qu'à la reconstitution (étape 662) d'au moins une cartographie correspondant à au moins un paramètre de caractérisation, à partir des éléments de cartographie enregistrés au cours du procédé.

La Fig. 6B illustre un schéma représentant un synoptique d'un quatrième exemple de procédé de caractérisation selon la présente description.

Dans cet exemple, les étapes similaires à celles illustrées au moyen de la Fig. 6A sont repérées par les mêmes références.

Dans cet exemple, le procédé ne comprend plus nécessairement d'étape de mosaïquage d'images de visée. Le procédé comprend ici une étape 643 de repérage de l'image de visée dans une image de surface de la région d'intérêt. Cette image de surface de la région d'intérêt peut être une image obtenue par un système indépendant, par exemple une image dermoscopique obtenue par un dermatoscope. Une étape 634 peut comprendre un affichage des éléments de cartographie reconstitués sur l'image de surface de la région d'intérêt. Bien que décrite à travers un certain nombre d'exemples de réalisation, les systèmes et procédés de caractérisation d'une région d'intérêt d'un tissu biologique selon la présente description comprend différentes variantes, modifications et perfectionnements qui apparaîtront de façon évidente à l'homme de l'art, étant entendu que ces différentes variantes, modifications et perfectionnements font partie de la portée de l'invention telle que définie par les revendications qui suivent.

### Références

Réf. 1 : A. Alfonso-Garcia et al. « Real-time augmented reality for delineation of surgical margins during neurosurgery using autofluorescence lifetime contrast", J Biophotonics, 2020 Jan;13(1):e201900108
Réf. 2 : WO2020087164
Réf. 3 : S. Li, "A review of feature detection and match algorithms for localization and mapping", IOP Conference Series: Materials Science and Engineering vol. 231 012003 (2017)
Réf. 4: Demande de brevet publiée WO2015092019
Réf. 5 : E. Beaurepaire et al. "Full-field optical coherence microscopy" Opt. Lett. 23, 244-246 (1998)
Réf. 6 : Ogien et al., "Video-mosaicking of human skin in vivo using handheld line-field confocal optical coherence tomography" Proc. SPIE 11211, Photonics in Dermatology and Plastic Surgery 2020, 1121114 (19 February 2020)
Réf. 7 : WO2022017784
Réf. 8 : FR 3107604
Réf. 9 : US9185357
Réf. 10 : A. Davis et al., « Line-field confocal optical coherence tomography operating simultaneously at 800 nm and 1300 nm center wavelengths", Progress in Biomedical Optics and Imaging, SPIE - International Society for Optical Engineering, Bellingham, WA, US, vol. 10890, 4 mars 2019, pages 1089021-1 - 1089021-7.

## Revendications

1. Système de caractérisation (101, 102, 200) d'une région d'intérêt (ROI) d'un tissu biologique (S), comprenant :
- un dispositif de visée (110) comprenant :
- un dispositif d'éclairage plein champ (111) configuré pour éclairer le tissu biologique dans une première bande spectrale ;
- un détecteur bidimensionnel (215) comprenant une aire de détection (216);
- un dispositif d'imagerie plein champ (211) comprenant un premier axe optique (Δ₁) et configuré pour conjuguer optiquement une surface élémentaire du tissu biologique avec ladite aire de détection du détecteur bidimensionnel, le détecteur bidimensionnel produisant, en fonctionnement, une image en réflexion de ladite surface élémentaire;
- un dispositif d'analyse microscopique (120) comprenant :
- un objectif de microscope (125) comprenant un deuxième axe optique (Δ₂), solidaire mécaniquement du premier axe optique;
- une voie d'éclairage configurée pour éclairer le tissu biologique selon un premier motif d'éclairage inclus dans ladite surface élémentaire, et dans une deuxième bande spectrale;
- une voie de détection comprenant ledit objectif de microscope, ladite voie de détection étant configurée pour détecter selon un motif de détection inclus dans ladite surface élémentaire, un faisceau lumineux émis par le tissu biologique en réponse audit éclairage du tissu biologique et générer une information d'analyse microscopique;
- une unité de traitement de données comprenant :
- un premier module de traitement (130) configuré pour déterminer à partir de ladite information d'analyse microscopique au moins un premier paramètre de caractérisation du tissu biologique en un nombre de points donné dudit motif de détection ;
- un deuxième module de traitement (140) configuré pour
- repérer, par rapport à une image de surface de la région d'intérêt, chaque image de surface élémentaire d' une pluralité d'images de surface élémentaires acquises successivement par déplacement du dispositif d'imagerie plein champ du dispositif de visée ; et
- produire au moins un premier élément de cartographie dudit au moins un premier paramètre de caractérisation, à partir dudit au moins un premier paramètre de caractérisation déterminé pour au moins une partie des images de surface élémentaires de la dite pluralité d'images de surface élémentaires;
- un module d'affichage (150) configuré pour afficher une cartographie du tissu comprenant au moins ledit premier élément de cartographie, ladite cartographie étant repérée par rapport à ladite image de surface de la région d'intérêt.

2. Système de caractérisation selon la revendication 1, dans lequel lesdites images de surface élémentaires présentent un recouvrement partiel et ladite image de surface de la région d'intérêt est produite par mosaïquage de ladite pluralité d'images de surface élémentaires.

3. Système de caractérisation selon la revendication 1, dans lequel ladite image de surface de la région d'intérêt est produite au moyen d'un système d'imagerie indépendant dudit système de caractérisation.

4. Système de caractérisation selon l'une quelconque des revendications précédentes, dans lequel le module d'affichage est configuré pour afficher en outre ladite image de surface de la région d'intérêt ou une autre image de surface de l'ensemble de la région d'intérêt acquise par un autre système d'imagerie et repérée par rapport à ladite image de surface de l'ensemble de la région d'intérêt.

5. Système de caractérisation selon la revendication 4, dans lequel le module d'affichage est configuré pour afficher ladite cartographie du tissu de façon superposée à ladite image de surface de la région d'intérêt ou à ladite autre image de surface de l'ensemble de la région d'intérêt.

6. Système de caractérisation selon l'une quelconque des revendications 4 ou 5, dans lequel le module d'affichage est configuré pour que ladite cartographie du tissu soit affichée de façon juxtaposée à ladite image de surface de la région d'intérêt ou à ladite autre image de surface de l'ensemble de la région d'intérêt, le module d'affichage étant configuré pour représenter en outre un curseur commun dans ladite cartographie du tissu et dans ladite image de surface de la région d'intérêt ou dans ladite cartographie du tissu et dans ladite autre image de surface de l'ensemble de la région d'intérêt.

7. Système de caractérisation selon l'une quelconque des revendications précédentes, dans lequel le dispositif d'imagerie plein champ du dispositif de visée comprend ledit objectif de microscope.

8. Système de caractérisation selon l'une quelconque des revendications précédentes, dans lequel ledit au moins un premier paramètre de caractérisation du tissu biologique comprend un paramètre choisi parmi : une mesure morphologique, une mesure cytologique, une mesure optique, une mesure caractérisant une composition chimique, une mesure mécanique, une combinaison de ces mesures, un score de caractérisation de l'état du tissu, par exemple basé sur l'une au moins de ces mesures.

9. Système de caractérisation selon l'une quelconque des revendications précédentes, dans lequel ledit premier module de traitement de ladite information d'analyse microscopique comprend un module d'intelligence artificielle basé sur un modèle d'apprentissage profond utilisant des réseaux de neurones.

10. Système de caractérisation selon l'une quelconque des revendications précédentes, dans lequel ledit dispositif d'analyse microscopique est configuré pour l'imagerie confocale et/ou l'imagerie tomographique par cohérence optique et ladite information d'analyse microscopique du tissu biologique comprend au moins une image du tissu biologique.

11. Système de caractérisation selon l'une quelconque des revendications 1 à 9, dans lequel ledit dispositif d'analyse microscopique est configuré pour l'analyse spectroscopique et ladite information d'analyse microscopique du tissu biologique comprend au moins un spectre dudit faisceau lumineux émis par le tissu biologique.

12. Système de caractérisation selon l'une quelconque des revendications précédentes, dans lequel le deuxième module de traitement est configuré, en outre, pour la détermination de marges d'exérèse d'une région à extraire dudit tissu biologique à partir dudit au moins un premier élément de cartographie.

13. Produit programme d'ordinateur comprenant des instructions de code de programme pour la mise en œuvre d'un procédé de traitement de données en vue de la caractérisation d'une région d'intérêt (ROI) d'un tissu biologique (S) , lorsque ledit programme est exécuté sur un ordinateur, les données étant obtenues au moyen d'un système de caractérisation comprenant :
- un dispositif de visée (110) comprenant :
- un dispositif d'éclairage plein champ (111) configuré pour éclairer le tissu biologique dans une première bande spectrale ;
- un détecteur bidimensionnel (215) comprenant une aire de détection (216);
- un dispositif d'imagerie plein champ (211) comprenant un premier axe optique (Δ₁) et configuré pour conjuguer optiquement une surface élémentaire du tissu biologique avec ladite aire de détection du détecteur bidimensionnel, le détecteur bidimensionnel produisant, en fonctionnement, une image en réflexion de ladite surface élémentaire;
- un dispositif d'analyse microscopique (120) comprenant :
- un objectif de microscope (125) comprenant un deuxième axe optique (Δ₂), solidaire mécaniquement du premier axe optique;
- une voie d'éclairage configurée pour éclairer le tissu biologique selon un premier motif d'éclairage inclus dans ladite surface élémentaire, et dans une deuxième bande spectrale;
- une voie de détection comprenant ledit objectif de microscope, ladite voie de détection étant configurée pour détecter selon un motif de détection inclus dans ladite surface élémentaire, un faisceau lumineux émis par le tissu biologique en réponse audit éclairage du tissu biologique et générer une information d'analyse microscopique;
le procédé de traitement de données comprenant les étapes suivantes:
- la détermination à partir de ladite information d'analyse microscopique dudit au moins un premier paramètre de caractérisation du tissu biologique en un nombre de points donné dudit motif de détection ;
- le repérage, par rapport à une image de surface de la région d'intérêt, de chaque image de surface élémentaire d' une pluralité d'images de surface élémentaires acquises successivement par déplacement du dispositif d'imagerie plein champ du dispositif de visée et
- la production d'au moins un premier élément de cartographie dudit au moins un premier paramètre de caractérisation, à partir dudit au moins un premier paramètre de caractérisation déterminé pour au moins une partie des images de surface élémentaires de la dite pluralité d'images de surface élémentaires.

14. Produit programme d'ordinateur selon la revendication 13 dans lequel :
- lesdites images de surface élémentaires présentent un recouvrement partiel et ladite image de surface de la région d'intérêt est produite par mosaïquage de ladite pluralité d'images de surface élémentaires.

15. Produit programme d'ordinateur selon l'une quelconque des revendications 13 ou 14, dans lequel la détermination dudit au moins un premier paramètre de caractérisation du tissu biologique est obtenue au moyen d'un module d'intelligence artificielle basé sur un modèle d'apprentissage profond utilisant des réseaux de neurones.

16. Moyen de stockage lisible par ordinateur et non transitoire, stockant un produit programme d'ordinateur selon l'une quelconque des revendications 13 à 15.

## Patentansprüche

1. System zur Charakterisierung (101, 102, 200) eines Interessenbereichs (ROI) eines biologischen Gewebes (S), beinhaltend:
- eine Zielvorrichtung (110), beinhaltend:
- eine Vollfeldbeleuchtungsvorrichtung (111), die dazu konfiguriert ist, das biologische Gewebe in einem ersten Spektralband zu beleuchten;
- einen zweidimensionalen Detektor (215), der eine Detektionsfläche (216) beinhaltet;
- eine Vollfeldbildgebungsvorrichtung (211), die eine erste optische Achse (Δ₁) beinhaltet und dazu konfiguriert ist, eine Elementaroberfläche des biologischen Gewebes mit der Detektionsfläche des zweidimensionalen Detektors optisch zu konjugieren, wobei der zweidimensionale Detektor im Betrieb ein reflektiertes Bild der Elementaroberfläche erzeugt;
- eine Vorrichtung zur mikroskopischen Analyse (120), beinhaltend:
- ein Mikroskopobjektiv (125), das eine zweite optische Achse (Δ₂) beinhaltet, die mit der ersten optischen Achse mechanisch fest verbunden ist;
- einen Beleuchtungsweg, der dazu konfiguriert ist, das biologische Gewebe gemäß einem ersten Beleuchtungsmuster, das in der Elementaroberfläche enthalten ist, und in einem zweiten Spektralband zu beleuchten;
- einen Detektionsweg, der das Mikroskopobjektiv beinhaltet, wobei der Detektionsweg dazu konfiguriert ist, gemäß einem Detektionsmuster, das in der Elementaroberfläche enthalten ist, einen Lichtstrahl zu detektieren, der von dem biologischen Gewebe als Reaktion auf die Beleuchtung des biologischen Gewebes emittiert wird, und eine Information einer mikroskopischen Analyse zu generieren;
- eine Datenverarbeitungseinheit, beinhaltend:
- ein erstes Verarbeitungsmodul (130), das dazu konfiguriert ist, anhand der Information der mikroskopischen Analyse mindestens einen ersten Parameter zur Charakterisierung des biologischen Gewebes an einer gegebenen Anzahl von Punkten des Detektionsmusters zu bestimmen;
- ein zweites Verarbeitungsmodul (140), das zu Folgendem konfiguriert ist:
- Markieren, in Bezug auf ein Oberflächenbild des Interessenbereichs, jedes Elementaroberflächenbildes einer Vielzahl von Elementaroberflächenbildern, die durch Bewegen der Vollfeldbildgebungsvorrichtung der Zielvorrichtung nacheinander erfasst werden; und
- Erzeugen mindestens eines ersten Kartographieelements des mindestens einen ersten Charakterisierungsparameters anhand des für mindestens einen Teil der Elementaroberflächenbilder der Vielzahl von Elementaroberflächenbildern bestimmten mindestens einen ersten Charakterisierungsparameters;
- ein Anzeigemodul (150), das dazu konfiguriert ist, eine Kartographie des Gewebes anzuzeigen, die mindestens das erste Kartographieelement beinhaltet, wobei die Kartographie in Bezug auf das Oberflächenbild des Interessenbereichs markiert ist.

2. System zur Charakterisierung nach Anspruch 1, wobei die Elementaroberflächenbilder eine teilweise Überlappung aufweisen und das Oberflächenbild des Interessenbereichs durch Mosaikierung der Vielzahl von Elementaroberflächenbildern erzeugt wird.

3. System zur Charakterisierung nach Anspruch 1, wobei das Oberflächenbild des Interessenbereichs mit Hilfe eines Bildgebungssystems erzeugt wird, das von dem Charakterisierungssystem unabhängig ist.

4. System zur Charakterisierung nach einem der vorhergehenden Ansprüche, wobei das Anzeigemodul dazu konfiguriert ist, ferner das Oberflächenbild des Interessenbereichs oder ein anderes Oberflächenbild des gesamten Interessenbereichs, das durch ein anderes Bildgebungssystem erfasst und in Bezug auf das Oberflächenbild des gesamten Interessenbereichs markiert ist, anzuzeigen.

5. System zur Charakterisierung nach Anspruch 4, wobei das Anzeigemodul dazu konfiguriert ist, die Kartographie des Gewebes so anzuzeigen, dass sie das Oberflächenbild des Interessenbereichs oder das andere Oberflächenbild des gesamten Interessenbereichs überlagert.

6. System zur Charakterisierung nach einem der Ansprüche 4 oder 5, wobei das Anzeigemodul dazu konfiguriert ist, dass die Kartographie des Gewebes neben dem Oberflächenbild des Interessenbereichs oder dem anderen Oberflächenbild des gesamten Interessenbereichs angezeigt wird, wobei das Anzeigemodul dazu konfiguriert ist, ferner einen gemeinsamen Cursor in der Kartographie des Gewebes und in dem Oberflächenbild des Interessenbereichs oder in der Kartographie des Gewebes und in dem anderen Oberflächenbild des gesamten Interessenbereichs darzustellen.

7. System zur Charakterisierung nach einem der vorhergehenden Ansprüche, wobei die Vollfeldbildgebungsvorrichtung der Zielvorrichtung das Mikroskopobjektiv beinhaltet.

8. System zur Charakterisierung nach einem der vorhergehenden Ansprüche, wobei der mindestens eine erste Parameter zur Charakterisierung des biologischen Gewebes einen Parameter beinhaltet, der aus Folgendem ausgewählt ist: einer morphologischen Messung, einer zytologischen Messung, einer optischen Messung, einer Messung, die eine chemische Zusammensetzung charakterisiert, einer mechanischen Messung, einer Kombination aus diesen Messungen, einem Score zur Charakterisierung des Zustands des Gewebes, der beispielsweise auf mindestens einer dieser Messungen basiert.

9. System zur Charakterisierung nach einem der vorhergehenden Ansprüche, wobei das erste Verarbeitungsmodul für die Information der mikroskopischen Analyse ein Modul künstlicher Intelligenz beinhaltet, das auf einem Deep-Learning-Modell basiert, das neuronale Netze verwendet.

10. System zur Charakterisierung nach einem der vorhergehenden Ansprüche, wobei die Vorrichtung zur mikroskopischen Analyse zur konfokalen Bildgebung und/oder zur Bildgebung mittels optischer Kohärenztomographie konfiguriert ist und die Information der mikroskopischen Analyse des biologischen Gewebes mindestens ein Bild des biologischen Gewebes beinhaltet.

11. System zur Charakterisierung nach einem der Ansprüche 1 bis 9, wobei die Vorrichtung zur mikroskopischen Analyse zur spektroskopischen Analyse konfiguriert ist und die Information der mikroskopischen Analyse des biologischen Gewebes mindestens ein Spektrum des von dem biologischen Gewebe emittierten Lichtstrahls beinhaltet.

12. System zur Charakterisierung nach einem der vorhergehenden Ansprüche, wobei das zweite Verarbeitungsmodul ferner zur Bestimmung von Absetzungsrändern eines zu entfernenden Bereichs des biologischen Gewebes anhand des mindestens einen ersten Kartographieelements konfiguriert ist.

13. Computerprogrammprodukt, das Programmcodeanweisungen beinhaltet, die bei Ausführung des Programms auf einem Computer ein Datenverarbeitungsverfahren mit dem Ziel der Charakterisierung eines Interessenbereichs (ROI) eines biologischen Gewebes (S) umsetzen, wobei die Daten mit Hilfe eines Charakterisierungssystems erhalten werden, das Folgendes beinhaltet:
- eine Zielvorrichtung (110), beinhaltend:
- eine Vollfeldbeleuchtungsvorrichtung (111), die dazu konfiguriert ist, das biologische Gewebe in einem ersten Spektralband zu beleuchten;
- einen zweidimensionalen Detektor (215), der eine Detektionsfläche (216) beinhaltet;
- eine Vollfeldbildgebungsvorrichtung (211), die eine erste optische Achse (Δ₁) beinhaltet und dazu konfiguriert ist, eine Elementaroberfläche des biologischen Gewebes mit der Detektionsfläche des zweidimensionalen Detektors optisch zu konjugieren, wobei der zweidimensionale Detektor im Betrieb ein reflektiertes Bild der Elementaroberfläche erzeugt;
- eine Vorrichtung zur mikroskopischen Analyse (120), beinhaltend:
- ein Mikroskopobjektiv (125), das eine zweite optische Achse (Δ₂) beinhaltet, die mit der ersten optischen Achse mechanisch fest verbunden ist;
- einen Beleuchtungsweg, der dazu konfiguriert ist, das biologische Gewebe gemäß einem ersten Beleuchtungsmuster, das in der Elementaroberfläche enthalten ist, und in einem zweiten Spektralband zu beleuchten;
- einen Detektionsweg, der das Mikroskopobjektiv beinhaltet, wobei der Detektionsweg dazu konfiguriert ist, gemäß einem Detektionsmuster, das in der Elementaroberfläche enthalten ist, einen Lichtstrahl zu detektieren, der von dem biologischen Gewebe als Reaktion auf die Beleuchtung des biologischen Gewebes emittiert wird, und eine Information einer mikroskopischen Analyse zu generieren; wobei das Datenverarbeitungsverfahren die folgenden Schritte beinhaltet:
- Bestimmen, anhand der Information der mikroskopischen Analyse, des mindestens einen ersten Parameters zur Charakterisierung des biologischen Gewebes an einer gegebenen Anzahl von Punkten des Detektionsmusters;
- Markieren, in Bezug auf ein Oberflächenbild des Interessenbereichs, jedes Elementaroberflächenbildes einer Vielzahl von Elementaroberflächenbildern, die durch Bewegen der Vollfeldbildgebungsvorrichtung der Zielvorrichtung nacheinander erfasst werden; und
- Erzeugen mindestens eines ersten Kartographieelements des mindestens einen ersten Charakterisierungsparameters anhand des für mindestens einen Teil der Elementaroberflächenbilder der Vielzahl von Elementaroberflächenbildern bestimmten mindestens einen ersten Charakterisierungsparameters.

14. Computerprogrammprodukt nach Anspruch 13, wobei:
- die Elementaroberflächenbilder eine teilweise Überlappung aufweisen und das Oberflächenbild des Interessenbereichs durch Mosaikierung der Vielzahl von Elementaroberflächenbildern erzeugt wird.

15. Computerprogrammprodukt nach einem der Ansprüche 13 oder 14, wobei das Bestimmen des mindesten einen ersten Parameters zur Charakterisierung des biologischen Gewebes mit Hilfe eines Moduls künstlicher Intelligenz erhalten wird, das auf einem Deep-Learning-Modul basiert, das neuronale Netze verwendet.

16. Computerlesbares und nicht transitorisches Speichermedium, das ein Computerprogrammprodukt nach einem der Ansprüche 13 bis 15 speichert.

## Claims

1. A system (101, 102, 200) for characterizing a region of interest (ROI) of a biological tissue (S), comprising:
- a sighting device (110) comprising:
- a full-field illumination device (111) configured to illuminate the biological tissue in a first spectral band;
- a two-dimensional detector (215) comprising a detection area (216);
- a full-field imaging device (211) comprising a first optical axis (Δ₁) and configured to optically conjugate an elementary surface of the biological tissue with said detection area of the two-dimensional detector, the two-dimensional detector producing, during operation, an image in reflection of said elementary surface;
- a microscopic analysis device (120) comprising:
- a microscope objective (125) comprising a second optical axis (Δ₂) mechanically integral with the first optical axis;
- an illumination path configured to illuminate the biological tissue in a first illumination pattern included in said elementary surface, and in a second spectral band;
- a detection path comprising said microscope objective, said detection path being configured to detect, in a detection pattern included in said elementary surface, a light beam emitted by the biological tissue in response to said illumination of the biological tissue and generate microscopic analysis information;
- a data processing unit comprising:
- a first processing module (130) configured to determine, from said microscopic analysis information, at least one first biological tissue characterization parameter at a given number of points of said detection pattern;
- a second processing module (140) configured to
- localize, relative to a surface image of the region of interest, each elementary surface image of a plurality of elementary surface images acquired successively by moving the full-field imaging device of the sighting device; and
- produce at least one first map element for said at least one first characterization parameter, from said at least one first characterization parameter determined for at least some of the elementary surface images of said plurality of elementary surface images;
- a display module (150) configured to display a tissue map comprising at least said first map element, said map being localized relative to said surface image of the region of interest.

2. The characterization system as claimed in claim 1, wherein said elementary surface images have a partial overlap and said surface image of the region of interest is produced by mosaicking said plurality of elementary surface images.

3. The characterization system as claimed in claim 1, wherein said surface image of the region of interest is produced by way of an imaging system independent of said characterization system.

4. The characterization system as claimed in any one of the preceding claims, wherein the display module is configured to additionally display said surface image of the region of interest or another surface image of the entire region of interest acquired by another imaging system and localized relative to said surface image of the entire region of interest.

5. The characterization system as claimed in claim 4, wherein the display module is configured to display said tissue map in a manner superimposed on said surface image of the region of interest or on said other surface image of the entire region of interest.

6. The characterization system as claimed in either one of claims 4 and 5, wherein the display module is configured such that said tissue map is displayed in a manner juxtaposed with said surface image of the region of interest or with said other surface image of the entire region of interest, the display module being configured to additionally show a common cursor in said tissue map and in said surface image of the region of interest or in said tissue map and in said other surface image of the entire region of interest.

7. The characterization system as claimed in any one of the preceding claims, wherein the full-field imaging device of the sighting device comprises said microscope objective.

8. The characterization system as claimed in any one of the preceding claims, wherein said at least one first biological tissue characterization parameter comprises a parameter chosen from among: a morphological measurement, a cytological measurement, an optical measurement, a measurement characterizing a chemical composition, a mechanical measurement, a combination of these measurements, a score for characterizing the state of the tissue, for example based on at least one of these measurements.

9. The characterization system as claimed in any one of the preceding claims, wherein said first module for processing said microscopic analysis information comprises an artificial intelligence module based on a deep learning model using neural networks.

10. The characterization system as claimed in any one of the preceding claims, wherein said microscopic analysis device is configured for confocal imaging and/or optical coherence tomography imaging and said biological tissue microscopic analysis information comprises at least one image of the biological tissue.

11. The characterization system as claimed in any one of claims 1 to 9, wherein said microscopic analysis device is configured for spectroscopic analysis and said biological tissue microscopic analysis information comprises at least one spectrum of said light beam emitted by the biological tissue.

12. The characterization system as claimed in any one of the preceding claims, wherein the second processing module is furthermore configured to determine excision margins of a region of said biological tissue to be extracted based on said at least one first map element.

13. A computer program product comprising program code instructions for implementing a data processing method with a view to characterizing a region of interest (ROI) of a biological tissue (S) when said program is executed on a computer, the data being obtained by way of a characterization system comprising:
- a sighting device (110) comprising:
- a full-field illumination device (111) configured to illuminate the biological tissue in a first spectral band;
- a two-dimensional detector (215) comprising a detection area (216);
- a full-field imaging device (211) comprising a first optical axis (Δ₁) and configured to optically conjugate an elementary surface of the biological tissue with said detection area of the two-dimensional detector, the two-dimensional detector producing, during operation, an image in reflection of said elementary surface;
- a microscopic analysis device (120) comprising:
- a microscope objective (125) comprising a second optical axis (Δ₂) mechanically integral with the first optical axis;
- an illumination path configured to illuminate the biological tissue in a first illumination pattern included in said elementary surface, and in a second spectral band;
- a detection path comprising said microscope objective, said detection path being configured to detect, in a detection pattern included in said elementary surface, a light beam emitted by the biological tissue in response to said illumination of the biological tissue and generate microscopic analysis information;
the data processing method comprising the following steps:
- determining, from said microscopic analysis information, said at least one first biological tissue characterization parameter at a given number of points of said detection pattern;
- localizing, relative to a surface image of the region of interest, each elementary surface image of a plurality of elementary surface images acquired successively by moving the full-field imaging device of the sighting device and
- producing at least one first map element for said at least one first characterization parameter, from said at least one first characterization parameter determined for at least some of the elementary surface images of said plurality of elementary surface images.

14. The computer program product as claimed in claim 13, wherein:
- said elementary surface images have a partial overlap and said surface image of the region of interest is produced by mosaicking said plurality of elementary surface images.

15. The computer program product as claimed in either one of claims 13 and 14, wherein said at least one first biological tissue characterization parameter is determined by way of an artificial intelligence module based on a deep learning model using neural networks.

16. A non-transient computer-readable storage medium storing a computer program product as claimed in any one of claims 13 to 15.
